(19) Europäisches Patentamt
European Patent Office
Office européen des brevets

(11) EP 1 934 172 B1

(12) EUROPÄISCHE PATENTSCHRIFT

(45) Veröffentlichungstag und Bekanntmachung des Hinweises auf die Patenterteilung:
**28.09.2011 Patentblatt 2011/39**

(21) Anmeldenummer: **06806896.4**

(22) Anmeldetag: **29.09.2006**

(51) Int Cl.:
***C07C 275/42*** *(2006.01)* ***C07C 273/18*** *(2006.01)*
***C07C 251/08*** *(2006.01)* ***C08G 18/32*** *(2006.01)*

(86) Internationale Anmeldenummer:
**PCT/EP2006/066925**

(87) Internationale Veröffentlichungsnummer:
**WO 2007/036572 (05.04.2007 Gazette 2007/14)**

(54) **ALDIMIN-HALTIGE VERBINDUNGEN**
COMPOUNDS CONTAINING ALDIMINE
COMPOSES CONTENANT DES ALDIMINES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **30.09.2005 EP 05109111**

(43) Veröffentlichungstag der Anmeldung:
**25.06.2008 Patentblatt 2008/26**

(73) Patentinhaber: **Sika Technology AG**
**6340 Baar (CH)**

(72) Erfinder: **BURCKHARDT, Urs**
**8049 Zürich (CH)**

(74) Vertreter: **Sika Patent Attorneys**
**C/o Sika Technology AG**
**Tüffenwies 16**
**Postfach**
**8048 Zürich (CH)**

(56) Entgegenhaltungen:
**EP-A- 1 384 709 EP-A- 1 544 204**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 934 172 B1

Printed by Jouve, 75001 PARIS (FR)

## Beschreibung

### Technisches Gebiet

Die Erfindung betrifft das Gebiet der Aldimine und Aldimin-haltigen Verbindungen.

### Stand der Technik

Aldimine sind Kondensationsprodukte aus Aminen und Aldehyden und stellen eine seit langem bekannte Stoffklasse dar. Bei Kontakt mit Wasser können Aldimine zu den entsprechenden Aminen und Aldehyden hydrolysieren, während sie in Abwesenheit von Wasser stabil sind. Aufgrund dieser Eigenart können sie als gebundene oder geschützte Form von Aminen, beziehungsweise Aldehyden, verwendet werden. So werden Aldimine beispielsweise in der Polyurethanchemie eingesetzt, wo sie als durch Feuchtigkeit aktivierbare Vernetzer, so genannte "latente Amine" oder "latente Härter", für Isocyanat-haltige Kunststoffvorläufer dienen. Die Verwendung eines Aldimins als latenten Härter in Isocyanat-haltigen Systemen hat dabei zweierlei Vorteile: zum einen lässt sich die Entstehung von unerwünschten Gasblasen im ausgehärteten Kunststoff vermeiden, da die Aushärtung über das latente Amin - im Gegensatz zur direkten Reaktion des Isocyanats mit Feuchtigkeit - nicht unter Freisetzung von Kohlendioxid ($CO_2$) verläuft; zum anderen lassen sich hohe Aushärtungsgeschwindigkeiten erzielen. Jedoch birgt die Verwendung eines Aldimins in einem lagerfähigen Isocyanat-haltigen Kunststoffvorläufer die Gefahr, dessen Lagerstabilität durch vorzeitige Reaktion zwischen Aldimino- und Isocyanatgruppen herabzusetzen. Beispielsweise werden in US 4,469,831, US 4,853,454 und US 5,087,661 Zusammensetzungen aus Polyisocyanaten und Polyaldiminen beschrieben, welche unter dem Einfluss von Feuchtigkeit zu hochmolekularen Kunststoffen vernetzen und damit aushärten. Derartige Polyaldimine spalten bei der Hydrolyse jedoch stark riechende Aldehyde ab. WO 2004/013088 A1 beschreibt geruchsfreie Polyaldimine, welche aus der Reaktion von primären Polyaminen und geruchsfreien Aldehyden hergestellt werden.

Aldimine, welche zusätzliche funktionelle Gruppen aufweisen, sind bekannt. US 4,224,417 beschreibt beispielsweise Hydroxyaldimine und ihre Umsetzungsprodukte mit Polyisocyanaten. US 3,493,543, US 3,554,974, US 4,108,842, US 4,404,379 und US 6,136,942 beschreiben Aminoaldimine, beziehungsweise Cycloaminale als tautomere Form davon, ihre Umsetzungsprodukte mit Polyisocyanaten und deren Verwendung als latente Härter für Isocyanat-haltige Zusammensetzungen, die unter dem Einfluss von Feuchtigkeit rasch und blasenfrei aushärten. Die in den genannten Schriften beschriebenen Zusammensetzungen haben aber den Nachteil, eine stark eingeschränkte Lagerstabilität zu besitzen. Dies rührt daher, dass die geschützten Aminogruppen, welche in Form von Aldimino- oder Cycloaminal-Gruppen in den beschriebenen Aldiminen oder ihren Umsetzungsprodukten enthalten sind, sich gegenüber Isocyanatgruppen nicht vollständig inert verhalten, sondern mit diesen, insbesondere den reaktiven aromatischen Isocyanatgruppen, auch in Abwesenheit von Feuchtigkeit schleichend reagieren und dadurch eine Viskositätserhöhung verursachen, welche die Zusammensetzung schon nach kurzer Zeit unbrauchbar machen kann. Ein weiterer Nachteil der beschriebenen Aldimine enthaltend einen aktiven Wasserstoff, sowie deren Umsetzungsprodukte und Zusammensetzungen daraus, besteht darin, dass sie bei Kontakt mit Feuchtigkeit, bedingt durch die bei der Hydrolyse der Aldiminogruppen freigesetzten, geruchsintensiven Aldehyde, eine starke Geruchsbildung zeigen und deshalb, besonders in Innenräumen, nur eingeschränkt anwendbar sind.

### Darstellung der Erfindung

Aufgabe der vorliegenden Erfindung ist es daher, Aldimine zur Verfügung zu stellen, die geruchsfrei sind, Aldehyde abspalten, welche ebenfalls geruchsfrei sind, sich insbesondere für Isocyanatgruppen aufweisende Kunststoffvorläufer einsetzen lassen, welche sich durch eine verbesserte Lagerstabilität auszeichnen.

Überraschenderweise hat sich gezeigt, dass Aldimin-haltige Verbindungen gemäss Anspruch 1 diese Aufgabe lösen. Es hat sich weiterhin gezeigt, dass diese Aldimin-haltigen Verbindungen Substanzen darstellen, welche über ausserordentliche Eigenschaften verfügen und welche sich als Kunststoffvorläufer oder als Bestandteil eines Kunststoffvorläufers einsetzen lassen. Sie lassen sich auf einfache Weise herstellen und weisen eine grosse Palette von Strukturmerkmalen und chemisch reaktiven Gruppen auf. Somit finden sie breite Einsatzmöglichkeiten in reaktiven Systemen. Isocyanat-haltige Zusammensetzungen, die unter Verwendung dieser Aldimin-haltigen Verbindungen hergestellt wurden, weisen eine gute Lagerstabilität auf. Solche Zusammensetzungen härten unter dem Einfluss von Feuchtigkeit rasch und ohne Blasenbildung aus, sind geruchsfrei und sind beispielsweise als Klebstoffe, Dichtstoffe, Beschichtungen oder Beläge mit guten mechanischen Eigenschaften geeignet.

### Wege zur Ausführung der Erfindung

Gegenstand der Erfindung sind Aldimin-haltige Verbindungen der Formel (I)

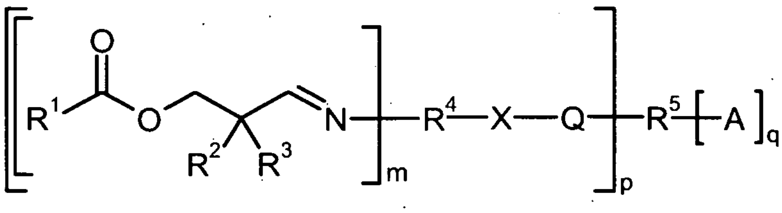

(I)

Hierbei steht m für eine ganze Zahl von 1 bis 4, p für eine ganze Zahl von 1 bis 6 und q für eine ganze Zahl von 0 bis 5, mit der Massgabe, dass die Summe von p und q einen Wert von 2 bis 6 darstellt. Weiterhin steht der Substituent $R^1$ entweder für einen einwertigen Kohlenwasserstoffrest mit 6 bis 30 C-Atomen, welcher gegebenenfalls mindestens ein Heteroatom, insbesondere in Form von Ether-Sauerstoff, aufweist, oder $R^1$ steht für einen Substituenten der Formel (II).

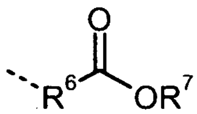

(II)

Hierbei steht der Substituent $R^6$ für einen zweiwertigen Kohlenwasserstoffrest mit 2 bis 20 C-Atomen, welcher gegebenenfalls mindestens ein Heteroatom, insbesondere in Form von Ether-Sauerstoff, aufweist. Der Substituent $R^7$ steht für einen einwertigen Kohlenwasserstoffrest mit 1 bis 20 C-Atomen.

Weiterhin stehen $R^2$ und $R^3$ entweder für zwei voneinander unabhängige Substituenten, die jeweils einen einwertigen Kohlenwasserstoffrest mit 1 bis 12 C-Atomen darstellen, oder $R^2$ und $R^3$ bilden zusammen einen einzigen Substituenten, der einen zweiwertigen Kohlenwasserstoffrest mit 4 bis 20 C-Atomen darstellt, welcher Teil eines carbocyclischen Rings mit 5 bis 8, bevorzugt 6, C-Atomen, ist, wobei dieser carbocyclische Ring gegebenenfalls substituiert ist.

Weiterhin steht der Substituent $R^4$ für einen (m+1)-wertigen Kohlenwasserstoffrest mit 2 bis 12 C-Atomen, welcher gegebenenfalls mindestens ein Heteroatom, insbesondere in Form von Ether-Sauerstoff oder tertiärem Amin-Stickstoff, enthält.

Des Weiteren steht X für O, S oder N-$R^8$, wobei hierbei $R^8$ entweder für einen einwertigen Kohlenwasserstoffrest mit 1 bis 20 C-Atomen, welche gegebenenfalls mindestens eine Carbonsäureester-, Nitril-, Nitro-, Phosphonsäureester-, Sulfon- oder Sulfonsäureestergruppe aufweist, steht, oder für einen Substituenten der Formel (III) steht.

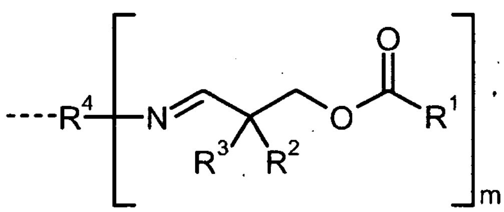

(III),

Weiterhin steht A für eine Reaktivgruppe, welche ausgewählt ist aus der Gruppe umfassend

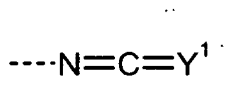 , 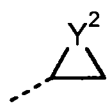 , 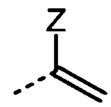 ,

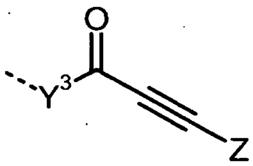 , 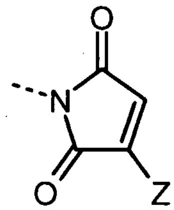 und 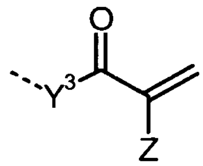 .

Hierbei stehen die Substituenten $Y^1$ für O oder S, $Y^2$ für O, S oder N-H, Z für H oder $CH_3$ und $Y^3$ für O oder N-H. Weiterhin steht $R^5$ entweder für einen (p+q)-wertigen organischen Rest. Ein derartiger Rest enthält gegebenenfalls Heteroatome und kann durch Entfernung von p+q Resten A aus $R^5$-$[A]_{p+q}$ erhalten werden. Oder der Substituent $R^5$ steht für N, $NR^{14}$, O, OC(O)O, Si, $P(O)0_3$ oder $SO_2$. Hierbei steht der Substituent $R^{14}$ für einen einwertigen Kohlenwasserstoffrest mit 1 bis 20 C-Atomen. Schliesslich steht Q für einen Substituenten, welcher ausgewählt ist aus der Gruppe umfassend

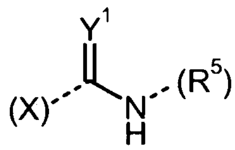 , 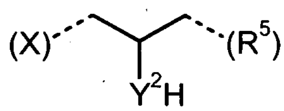 , 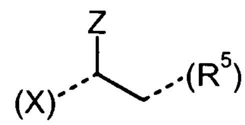 ,

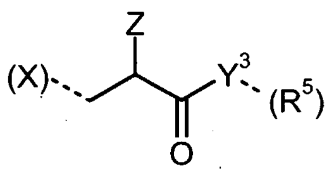 , 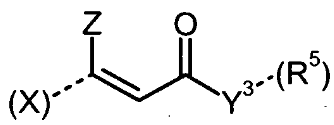 und 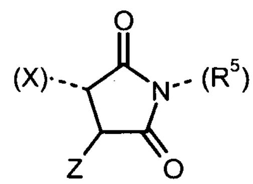

Die gestrichelten Linien in den Formeln stellen die Bindungen zu den angebenen Substituenten dar.

Die Aldimin-haltige Verbindung der Formel (I) ist herstellbar durch die Umsetzung von mindestens einem Aldimin der Formel (XI) enthaltend einen aktiven Wasserstoff mit mindestens einer Verbindung **D,** die mehr als eine Reaktivgruppe trägt, welche mit der Gruppe XH Additionsreaktionen eingehen kann. Die den aktiven Wasserstoff tragende Reaktivgruppe XH des Aldimins der Formel (XI) reagiert dabei in einer Additionsreaktion mit einer oder mehreren Reaktivgruppen der Verbindung **D** zu einer im Folgenden auch "Additionsprodukt" genannten Aldimin-haltigen Verbindung. Der Begriff "aktiver Wasserstoff' bezeichnet im vorliegenden Dokument ein deprotonierbares, an ein Stickstoff-, Sauerstoff- oder Schwefelatom gebundenes Wasserstoffatom. Der Begriff "Reaktivgruppe enthaltend einen aktiven Wasserstoff' bezeichnet eine einen aktiven Wasserstoff aufweisende funktionelle Gruppe, insbesondere eine primäre oder sekundäre Aminogruppe, eine Hydroxylgruppe, eine Mercaptogruppe oder eine Harnstoffgruppe. Attribute von Substanzen wie "Aldimin-haltig" oder "Isocyanathaltig" weisen darauf hin, dass die bezeichneten funktionellen Gruppen, also Aldiminogruppen oder Isocyanatgruppen, in den Substanzen enthalten sind.

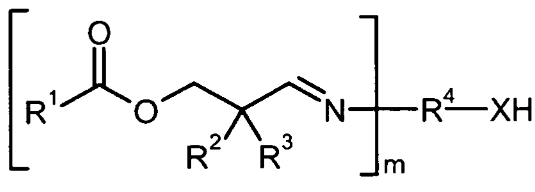 (XI)

Die Substituenten $R^1$, $R^2$, $R^3$, $R^4$ und X sowie der Index m haben die bereits für die Aldimin-haltige Verbindung der

Formel (I) offenbarten Bedeutungen.

Das Aldimin der Formel (XI) ist herstellbar aus mindestens einem sterisch gehinderten aliphatischen Aldehyd **A** und mindestens einem aliphatischen Amin **B,** entsprechend der Formel $[H_2N]_m$-$R^4$-XH, welches neben einer oder mehreren primären Aminogruppen noch eine weitere Reaktivgruppe enthaltend einen aktiven Wasserstoff aufweist.

Die Umsetzung zwischen dem Aldehyd **A** und dem Amin **B** erfolgt in einer Kondensationsreaktion unter Abspaltung von Wasser. Solche Kondensationsreaktionen sind bestens bekannt und beschrieben, beispielsweise in Houben-Weyl, "Methoden der organischen Chemie", Vol. XI/2, Seite 73ff. Der Aldehyd **A** wird hierbei in Bezug auf die primären Aminogruppen des Amins **B** stöchiometrisch oder in stöchiometrischem Überschuss eingesetzt. Üblicherweise werden solche Kondensationsreaktionen in Anwesenheit eines Lösemittels durchgeführt, mittels welchem das bei der Reaktion entstehende Wasser azeotrop entfernt wird. Zur Herstellung der Aldimine der Formel (XI) wird jedoch ein Herstellverfahren ohne Verwendung von Lösemitteln bevorzugt, wobei das bei der Kondensation gebildete Wasser direkt mittels Anlegen von Vakuum aus der Reaktionsmischung entfernt wird. Durch die lösemittelfreie Herstellung erübrigt sich ein Abdestillieren des Lösemittels nach erfolgter Herstellung, was den Herstellungsprozess vereinfacht. Zudem ist das Aldimin so frei von Lösemittelrückständen, welche einen störenden Geruch verursachen könnten.

Für die Herstellung des Aldimins der Formel (XI) wird mindestens ein sterisch gehinderter aliphatischer Aldehyd **A** der Formel (IV) eingesetzt.

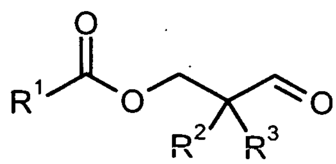
(IV)

In der Formel (IV) haben $R^1$, $R^2$ und $R^3$ die gleiche Bedeutung wie für Formel (I) beschrieben.

Der Aldehyd **A** ist geruchsfrei. Unter einer "geruchsfreien" Substanz wird eine Substanz verstanden, die so geruchsarm ist, dass sie für die meisten menschlichen Individuen nicht riechbar, das heisst mit der Nase nicht wahrnehmbar, ist.

Der Aldehyd **A** wird beispielsweise hergestellt aus einer Carbonsäure $R^1$-COOH und einem β-Hydroxyaldehyd der Formel (V) in einer Veresterungsreaktion. Diese Veresterung kann nach bekannten Methoden erfolgen, beschrieben beispielsweise in Houben-Weyl, "Methoden der organischen Chemie", Vol. VIII, Seiten 516-528. Der β-Hydroxyaldehyd der Formel (V) wird beispielsweise in einer gekreuzten Aldol-Addition aus Formaldehyd (oder oligomeren Formen von Formaldehyd, wie Paraformaldehyd oder 1,3,5-Trioxan) und einem Aldehyd der Formel (VI) erhalten.

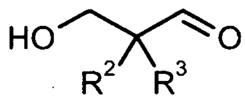
(V)

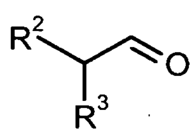
(VI)

In den Formeln (V) und (VI) haben $R^2$ und $R^3$ die gleiche Bedeutung wie für Formel (I) beschrieben.

Die Herstellung des Aldehyds **A** erfolgt bevorzugt lösemittelfrei. Dabei wird der β-Hydroxyaldehyd der Formel (V) ohne Verwendung von Lösemitteln direkt mit der Carbonsäure umgesetzt, wobei das bei der Veresterung gebildete Wasser im Vakuum entfernt wird. Es ist weiterhin bevorzugt, die zum Aldehyd **A** führenden Aldol- und Veresterungsreaktionen aus den Grundstoffen in einem gemeinsamen Prozessschritt, als Eintopfreaktion, auszuführen.

Als geeignete Carbonsäuren $R^1$-COOH zur Veresterung mit den β-Hydroxyaldehyden der Formel (V) seien beispielsweise die folgenden erwähnt: gesättigte aliphatische Carbonsäuren wie Oenanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure; einfach ungesättigte aliphatische Carbonsäuren wie Palmitoleinsäure, Ölsäure, Erucasäure; mehrfach ungesättigte aliphatische Carbonsäuren wie Linolsäure, Linolensäure, Elaeostearinsäure, Arachidonsäure; cycloaliphatische Carbonsäuren wie Cyclohexancarbonsäure; arylaliphatische Carbonsäuren wie Phenylessigsäure; aromatische Carbonsäuren wie Benzoesäure, Naphthoesäure, Toluylsäure, Anissäure; Isomere dieser Säuren; Fettsäuregemische aus der technischen Verseifung von natürlichen Ölen und Fetten wie beispielsweise Rapsöl, Sonnenblumenöl, Leinöl, Olbaumöl, Kokosnussöl, Ölpalmkernöl und Ölpalmöl; sowie Dicarbonsäuremonoalkyl- und -arylester, wie sie aus der einfachen Veresterung von Dicarbonsäuren wie Bernsteinsäure, Glutarsäure, Adipinsäure, Pimelinsäure, Korksäure, Azelainsäure, Sebacinsäure, 1,12-Dodecandisäure, Maleinsäure, Fumarsäure, Hexahydrophthalsäure, Hexahydroisophthalsäure, Hexahydroterephthalsäure, 3,6,9-Trioxaundecandisäure und ähnliche Derivate von Polyethylenglykol, mit Alkoholen wie Methanol, Ethanol, Propanol, Butanol, höheren Homologen und Isomeren dieser Alkohole erhalten werden.

Bevorzugt sind Caprylsäure, Caprinsäure, Laurinsäure, Myristinsäure, Palmitinsäure, Stearinsäure, Ölsäure, Linolsäure, Linolensäure, die Isomeren dieser Säuren sowie technische Gemische von Fettsäuren, welche diese Säuren enthalten. Besonders bevorzugt ist Laurinsäure.

Geeignete Aldehyde der Formel (VI) zur Umsetzung mit Formaldehyd zu β-Hydroxyaldehyden der Formel (V) sind beispielsweise Isobutyraldehyd, 2-Methylbutyraldehyd; 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd und Diphenylacetaldehyd. Bevorzugt ist Isobutyraldehyd.

Geeignete β-Hydroxyaldehyde der Formel (V) sind beispielsweise die Produkte aus der Umsetzung von Formaldehyd mit den vorgängig als geeignet genannten Aldehyden der Formel (VI). Bevorzugt ist 3-Hydroxypivalaldehyd.

Das Amin **B** ist ein aliphatisches Amin, welches neben einer oder mehreren primären Aminogruppen noch eine weitere Reaktivgruppe, welche einen aktiven Wasserstoff enthält, aufweist. Der Begriff "primäre Aminogruppe" bezeichnet im vorliegenden Dokument eine $NH_2$-Gruppe, die an einen organischen Rest gebunden ist, während der Begriff "sekundäre Aminogruppe" eine NH-Gruppe bezeichnet, die an zwei organische Reste gebunden ist. Der Begriff "aliphatisches Amin" bezeichnet Verbindungen, die mindestens eine Aminogruppe enthalten, die an einen aliphatischen, cycloaliphatischen oder arylaliphatischen Rest gebunden ist. Sie unterscheiden sich damit von den aromatischen Aminen, in welchen die Aminogruppe direkt an einen aromatischen Rest gebunden ist, wie beispielsweise in Anilin oder 2-Aminopyridin.

Als Amine **B** eignen sich beispielsweise die im Folgenden genannten Verbindungen:

- aliphatische Hydroxyamine wie 2-Aminoethanol, 2-Methylaminoethanol, 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol; eine primäre Aminogruppe tragende Derivate von Glykolen wie Diethylenglykol, Dipropylenglykol, Dibutylenglykol und höheren Oligomeren und Polymeren dieser Glykole, beispielsweise 2-(2-Aminoethoxy)-ethanol, Triethylenglykol-monoamin, α-(2-Hydroxymethylethyl)-ω-(2-amino-methylethoxy)-poly(oxy(methyl-1,2-ethandiyl)); eine Hydroxylgruppe und eine oder mehrere primäre Aminogruppen tragende Derivate von polyalkoxylierten drei- oder höherwertigen Alkoholen oder von polyalkoxylierten Diaminen; Produkte aus der einfachen Cyanoethylierung und anschliessender Hydrierung von Glykolen, beispielsweise 3-(2-Hydroxyethoxy)-propylamin, 3-(2-(2-Hydroxyethoxy)-ethoxy)-propylamin, 3-(6-Hydroxyhexyloxy)-propylamin;
- aliphatische Mercaptoamine wie 2-Aminoethanthiol (Cysteamin), 3-Aminopropanthiol, 4-Amino-1-butanthiol, 6-Amino-1-hexanthiol, 8-Amino-1-octanthiol, 10-Amino-1-decanthiol, 12-Amino-1-dodecanthiol; Aminothiozucker wie 2-Amino-2-deoxy-6-thioglucose;
- zwei- oder mehrwertige aliphatische Amine, welche neben einer oder mehreren primären Aminogruppen eine sekundäre Aminogruppe tragen, wie N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, 3-(4-Aminobutyl)-piperidin, N-Aminoethyl-piperazin, Diethylentriamin (DETA), Bis-hexamethylentriamin (BHMT); Di- und Triamine aus der Cyanoethylierung oder Cyanobutylierung von primären Mono- und Diaminen, beispielsweise N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methylamino-1-pentylamin,3-Ethylamino-1-pentylamin,3-Butyl-amino-1-pentylamin, 3-Hexylamino-1-pentylamin, 3-(2-Ethylhexyl)amino-1-pentylamin,3-Dodecylamino-1-pentylamin,3-Cyclohexylamino-1-pentylamin, Dipropylentriamin (DPTA), N3-(3-Aminopentyl)-1,3-pentandiamin, N5-(3-Amino-propyl)-2-methyl-1,5-pentandiamin, N5-(3-Amino-1-ethylpropyl)-2-methyl-1,5-pentandiamin,und Fettdiamine wie N-Cocoalkyl-1,3-propandiamin, N-Oleyl-1,3-propandiamin, N-Soyaalkyl-1,3-propandiamin, N-Talgalkyl-1,3-propandiamin oder N-($C_{16-22}$-Alkyl)-1,3-propandiamin, wie sie beispielsweise unter dem Handelsnamen Duomeen® von Akzo Nobel erhältlich sind; die Produkte aus der Michael-artigen Addition von aliphatischen primären Di- oder Polyaminen mit Acrylnitril, Malein- oder Fumarsäurediestern, Citraconsäurediestern, Acryl- und Methacrylsäureestern und Itaconsäurediestern, umgesetzt im Molverhältnis 1:1;
- trisubstituierte Harnstoffe, welche eine oder mehrere primäre Aminogruppen tragen, wie N-(2-Aminoethyl)-ethylenharnstoff, N-(2-Aminoethyl)-propylenharnstoff oder N-(2-Aminoethyl)-N'-methylharnstoff.

Insbesondere geeignete aliphatische Hydroxy- und Mercaptoamine sind solche, in denen die primäre Aminogruppe von der Hydroxyl- beziehungsweise der Mercaptogruppe durch eine Kette von mindestens 5 Atomen, oder durch einen Ring, getrennt sind, wie beispielsweise in 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin, $\alpha$-(2-Hydroxymethylethyl)-$\omega$-(2-aminomethylethoxy)-poly(oxy(methyl-1,2-ethandiyl)), 3-(2-Hydroxyethoxy)-propylamin, 3-(2-(2-Hydroxyethoxy)-ethoxy)-propylamin, 3-(6-Hydroxyhexyloxy)-propylamin, 6-Amino-1-hexanthiol, 8-Amino-1-octanthiol, 10-Amino-1-decanthiol und 12-Amino-1-dodecanthiol.

Ais Amine **B** bevorzugt sind zwei- oder mehrwertige aliphatische Amine, welche neben einer oder mehreren primären Aminogruppen eine sekundäre Aminogruppe tragen, insbesondere N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT und Fettdiamine wie N-Cocoalkyl-1,3-propandiamin, N-Oleyl-1,3-propandiamin, N-Soyaalkyl-1,3-propändiamin und N-Talgalkyl-1,3-propandiamin. Bevorzugt sind auch aliphatische Hydroxy- und Mercaptoamine, in denen die primäre Aminogruppe von der Hydroxy- beziehungsweise der Mercaptogruppe durch eine Kette von mindestens 5 Atomen, oder durch einen Ring, getrennt sind, insbesondere 5-Amino-1-pentanol, 6-Amino-1-hexanol und höhere Homologe davon, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin und höhere Oligo- und Polymere davon, 3-(2-Hydroxy-ethoxy)-propylamin, 3-(2-(2-Hydroxyethoxy)-ethoxy)-propylamin sowie 3-(6-Hydroxyhexyloxy)-propylamin.

Die Umsetzung zwischen einem Aldehyd **A** und einem Amin **B** führt dabei zu Hydroxyaldiminen, wenn als Amin **B** ein Hydroxyamin eingesetzt wird; zu Mercaptoaldiminen, wenn als Amin **B** ein Mercaptoamin eingesetzt wird; zu Aminoaldiminen, wenn als Amin **B** ein zwei- oder mehrwertiges Amin, welches neben einer oder mehreren primären Aminogruppen eine oder mehrere sekundäre Aminogruppen trägt, eingesetzt wird; oder zu Harnstoffaldiminen, wenn als Amin **B** ein trisubstituierter Harnstoff, welcher eine oder mehrere primäre Aminogruppen trägt, eingesetzt wird.

In einer Ausführungsform weisen die Aldimine der Formel (XI) einen Substituenten N-$R^8$ als Substituenten X auf. Derartige Aldimine der Formel (XI) lassen sich dadurch herstellen; dass mindestens ein sterisch gehinderter aliphatischer Aldehyd **A** der Formel (IV) mit mindestens einem zwei- oder mehrwertigen aliphatischen primären Amin **C** der Formel $[H_2N]_m$-$R^4$-$NH_2$ in einem ersten Schritt zu einem Zwischenprodukt der Formel (VII) umgesetzt wird, welches neben einer oder mehreren Aldiminogruppen noch eine primäre Aminogruppe enthält, und dieses Zwischenprodukt anschliessend in einem zweiten Schritt in einer Additionsreaktion mit einem Michaei-Akzeptor der Formel (VIII) in einem Verhältnis der Anzahl Doppelbindungen : Anzahl $NH_2$-Gruppen = 1:1 umgesetzt wird. Dabei entsteht ein Aminoaldimin, welches neben einer oder mehreren Aldiminogruppen noch.mindestens eine, bevorzugt genau eine, sekundäre Aminogruppe enthält.

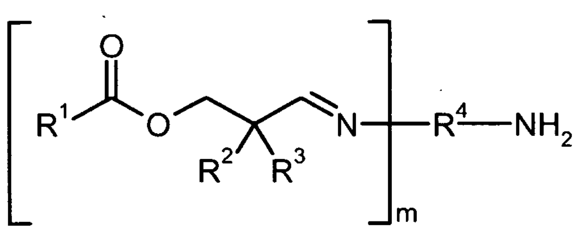
(VII)

In der Formel (VII) haben m, $R^1$, $R^2$, $R^3$ und $R^4$ die gleiche Bedeutung wie für Formel (I) beschrieben.

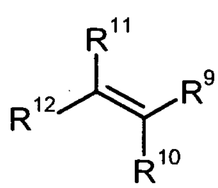
(VIII)

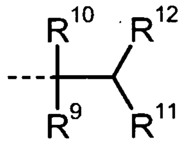 (IX)   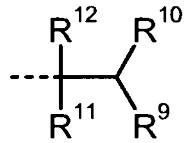 (IX')

Somit entstehen Aldimine der Formel (XI), bei welchen X für den Resten N-$R^8$ steht, und $R^8$ ein einwertiger Kohlenwasserstoffrest der Formel (IX) oder (IX') darstellt. In den Formeln (VIII), (IX) und (IX') stehen hierbei $R^9$ für einen Rest, welcher ausgewählt ist aus der Gruppe bestehend aus $-COOR^{13}$, -CN, $-NO_2$, $-PO(OR^{13})_2$, $-SO_2R^{13}$ und $-SO_2OR^{13}$ und $R^{10}$ für ein Wasserstoffatom oder einen Rest aus der Gruppe bestehend aus $-R^{13}$, $-COOR^{13}$ und $-CH_2COOR^{13}$ und $R^{11}$ und $R^{12}$ unabhängig voneinander für ein Wasserstoffatom oder einen Rest aus der Gruppe bestehend aus $-R^{13}$, $-COOR^{13}$ und -CN, wobei $R^{13}$ für einen einwertigen Kohlenwasserstoffrest mit 1 bis 20 C-Atomen steht.

Das Amin **C** ist ein aliphatisches Amin mit mindestens zwei primären Aminogruppen.

Beispiele für geeignete Amine **C** sind aliphatische Polyamine wie Ethylendiamin, 1,2- und 1,3-Propandiamin, 2-Methyl-1,2-propandiamin, 2,2-Dimethyl-1,3-propandiamin, 1,3- und 1,4-Butandiamin, 1,3- und 1,5-Pentandiamin, 2-Butyl-2-ethyl-1,5-pentandiamin, 1,6-Hexamethylendiamin (HMDA), 2,2,4- und 2,4,4-Trimethylhexamethylendiamin und Mischungen davon (TMD), 1,7-Heptandiamin, 1,8-Octandiamin, 2,4-Dimethyl-1,8-octandiamin, 4-Aminomethyl-1,8-octandiamin, 1,9-Nonandiamin, 2-Methyl-1,9-nonandiamin, 5-Methyl-1,9-nonandiamin, 1,10-Decandiamin, Isodecandiamin, 1,11-Undecandiamin, 1,12-Dodecandiamin, Methyl-bis-(3-aminopropyl)amin, 1,5-Diamino-2-methylpentan (MPMD), 1,3-Diaminopentan(DAMP), 2,5-Dimethyl-1,6-hexamethylendiamin; cycloaliphatische Polyamine wie 1,2-, 1,3- und 1,4-Diaminocyclohexan, Bis-(4-aminocyclohexyl)-methan ($H_{12}MDA$), Bis-(4-amino-3-methylcyclohexyl)-methan, Bis-(4-amino-3-ethylcyclohexyl)-methan, Bis-(4-amino-3,5-dimethylcyclohexyl)-methan, Bis-(4-amino-3-ethyl-5-methylcyclohexyl)-methan (M-MECA), 1-Amino-3-aminomethyl-3,5,5-trimethylcyclohexan (= Isophorondiamin oder IPDA), 2-und 4-Methyl-1,3-diaminocyclohexan und Mischungen davon, 1,3- und 1,4-Bis-(aminomethyl)cyclohexan, 1,3,5-Tris-(aminomethyl)cyclohexan, 1-Cyclohexylamino-3-aminopropan, 2,5(2,6)-Bis-(aminomethyl)-bicyclo[2.2.1]heptan (NBDA, hergestellt von Mitsui Chemicals), 3(4),8(9)-Bis-(aminomethyl)-tricyclo-[5.2.1.$0^{2,6}$]decan, 1,4-Diamino-2,2,6-trimethylcyclohexan (TMCDA), 3,9-Bis-(3-aminopropyl)-2,4,8,10-tetraoxaspiro[5.5]undecan; arylaliphatische Polyamine wie 1,3-Xylylendiamin (MXDA), 1,4-Xylylendiamin (PXDA), 1,3,5-Tris-(aminomethyl)benzol; Ethergruppen-haltige aliphatische Polyamine wie Bis-(2-aminoethyl)ether, 4,7-Dioxadecan-1,10-diamin, 4,9-Dioxadodecan-1,12-diamin und höhere Oligomere davon; Polyoxyalkylen-Polyamine mit theoretisch zwei oder drei Aminogruppen, erhältlich beispielsweise unter dem Namen Jeffamine® (hergestellt von Huntsman Chemicals). Bevorzugt sind Di- oder Triamine, in denen die primären Aminogruppen durch eine Kette von mindestens 5 Atomen, oder durch einen Ring, getrennt sind, insbesondere 1,5-Diamino-2-methylpentan, 1,6-Hexamethylendiamin, 2,2,4- und 2,4,4-Trimethylhexamethylendiamin und Mischungen davon, 1,10-Decandiamin, 1,12-Dodecandiamin, 1,3- und 1,4-Diaminocyclohexan, Bis-(4-aminocyclohexyl)-methan, Bis-(4-amino-3-methylcyclohexyl)-methan, 1-Amino-3-aminomethyl-3,5,5-trimethylcyclohexan, 1,3- und 1,4-Bis-(aminomethyl)cyclohexan, 2,5(2,6)-Bis-(aminomethyl)-bicyclo[2.2.1]heptan, 3(4),8(9)-Bis-(aminomethyl)-tricyclo-[5.2.1.$0^{2,6}$]decan, 1,4-Diamino-2,2,6-trimethylcyclohexan (TMCDA), 3,9-Bis-(3-aminopropyl)-2,4,8,10-tetraoxaspiro[5.5]undecan, 1,3- und 1,4-Xylylendiamin, 1,3,5-Tris-(aminomethyl)benzol sowie Polyoxyalkylen-Polyamine mit theoretisch zwei oder drei Aminogruppen, erhältlich beispielsweise unter dem Namen Jeffamine® (hergestellt von Huntsman Chemicals).

Beispiele für geeignete Michael-Akzeptoren der Formel (VIII) sind Malein- oder Fumarsäurediester wie Dimethylmaleinat, Diethylmaleinat, Dibutylmaleinat, Diethylfumarat; Citraconsäurediester wie Dimethylcitraconat; Acryl- oder Methacrylsäureester wie Methyl(meth)acrylat, Ethyl(meth)acrylat, Butyl(meth)acrylat, Lauryl(meth)acrylat, Stearyl(meth)acrylat, Tetrahydrofuryl-(meth)acrylat, Isobornyl(meth)acrylat; Itaconsäurediester wie Dimethylitaconat; Zimtsäureester wie Methylcinnamat; Vinylphosphonsäurediester wie Vinylphosphonsäuredimethylester; Vinylsulfonsäureester, insbesondere Vinylsulfonsäurearylester; Vinylsulfone; Vinylnitrile wie Acrylnitril, 2-Pentennitril oder Fumaronitril; 1-Nitroethylene wie β-Nitrostyrol; und Knoevenagel-Kondensationsprodukte, wie beispielsweise solche aus Malonsäurediestern und Aldehyden wie Formaldehyd, Acetaldehyd oder Benzaldehyd. Bevorzugt sind Maleinsäurediester, Acrylsäureester, Phosphonsäurediester und Vinylnitrile.

Die Umsetzung des Aldehyds **A** mit dem Amin **C** zum Zwischenprodukt der Formel (VII) erfolgt in einer Kondensationsreaktion unter Abspaltung von Wasser, wie sie weiter oben für die Umsetzung des Aldehyds **A** mit dem Amin **B** beschrieben wird. Die Stöchiometrie zwischen dem Aldehyd **A** und dem Amin **C** wird dabei so gewählt, dass m mol Aldehyd **A** für 1 mol Amin **C,** welches m+1 primäre Aminogruppen enthält, eingesetzt werden. Es wird ein lösemittelfreies Herstellverfahren bevorzugt, wobei das bei der Kondensation gebildete Wasser mittels Anlegen von Vakuum aus der Reaktionsmischung entfernt wird.

Die Umsetzung des Zwischenprodukts der Formel (VII) mit dem Michael-Akzeptor der Formel (VIII) erfolgt beispielsweise dadurch, dass das Zwischenprodukt mit einer stöchiometrischen oder leicht überstöchiometrischen Menge des Michael-Akzeptors der Formel (VIII) gemischt wird und die Mischung bei Temperaturen von 20 bis 110°C bis zum vollständigen Umsatz des Zwischenprodukts zum Aldimin der Formel (XI) erwärmt wird. Die Umsetzung erfolgt bevorzugt ohne eine Verwendung von Lösemitteln.

Die Aldimine der Formel (XI) können gegebenenfalls im Gleichgewicht stehen mit cyclischen Formen, wie sie in Formel (X) beispielhaft gezeigt sind. Diese cyclischen Formen sind im Fall von Aminoaldiminen cyclische Aminale, beispielsweise Imidazolidine oder Tetrahydropyrimidine; im Fall von Hydroxyaldiminen cyclische Aminoacetale, beispielsweise Oxazolidine oder Tetrahydrooxazine; im Fall von Mercaptoaldiminen cyclische Thioaminale, beispielsweise Thiazolidine oder Tetrahydrothiazine.

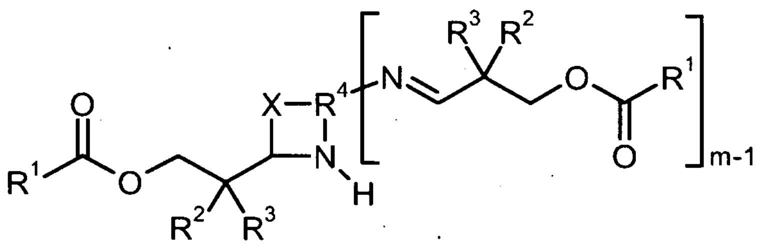 (X)

In der Formel (X) haben m, $R^1$, $R^2$, $R^3$, $R^4$ und X die gleiche Bedeutung wie für Formel (I) beschrieben.

Überraschenderweise neigen die meisten Aldimine der Formel (XI) nicht zur Cyclisierung. Insbesondere für Aminoaldimine kann mittels IR- und NMR-spektroskopischen Methoden gezeigt werden, dass diese Verbindungen überwiegend in der offenkettigen, also der Aldimin-Form, vorliegen, während die cyclische, also die Aminal-Form, nicht oder nur in Spuren vorkommt. Dies steht im Gegensatz zum Verhalten der Aminoaldimine nach dem Stand der Technik, wie sie beispielsweise in US 4,404,379 und US 6,136,942 beschrieben werden: jene liegen nämlich hauptsächlich in der Cycloaminal-Form vor. Auch Hydroxy- und Mercaptoamine, in denen die primäre Aminogruppe von der Hydroxy- beziehungsweise der Mercaptogruppe durch eine Kette von mindestens 5 Atomen, oder durch einen Ring, getrennt sind, zeigen kaum Cyclisierung. Die weitgehende Abwesenheit cyclischer Strukturen in den Aldiminen der Formel (XI) ist, insbesondere im Hinblick auf deren Verwendung in Isocyanat-haltigen Zusammensetzungen, als Vorteil zu werten, da die Aldimine dadurch weitgehend frei sind von den in Aminalen, Oxazolidinen und Thioaminalen vorkommenden basischen Stickstoffatomen, welche die Lagerstabilität der Isocyanat-haltigen Zusammensetzung herabsetzen könnten.

Die Aldimine der Formel (XI) sind geruchsfrei. Sie sind unter geeigneten Bedingungen, insbesondere unter Ausschluss von Feuchtigkeit, lagerstabil. Bei Zutritt von Feuchtigkeit können die Aldiminogruppen der Aldimine über Zwischenstufen formal zu Aminogruppen hydrolysieren, wobei der entsprechende, zur Herstellung des Aldimins verwendete Aldehyd **A** freigesetzt wird. Da diese Hydrolysereaktion reversibel ist und das chemische Gleichgewicht deutlich auf der Aldiminseite liegt, ist davon auszugehen, dass in Abwesenheit von gegenüber Aminen reaktiven Gruppen nur ein Teil der Aldiminogruppen teilweise oder ganz hydrolysieren.

Als Verbindungen **D** geeignet sind Substanzen, welche mehr als eine der folgenden Reaktivgruppen, welche Additionsreaktionen eingehen können, tragen: Isocyanat-, Isothiocyanat-, Cyclocarbonat-, Epoxid-, Episulfid-, Aziridin-, Acryl-, Methacryl-, 1-Ethinylcarbonyl-, 1-Propinylcarbonyl-, Maleimid-, Citraconimid-, Vinyl-, Isopropenyl- und Allyl-Gruppen, sowie Verbindungen mit verschiedenen der vorgenannten Reaktivgruppen. Bevorzugt sind Isocyanat-, Epoxid-, Acryl-, Maleimid-, Vinyl-, Isopropenyl- und Allylgruppen. Besonders bevorzugt ist die Isocyanatgruppe.

Als Verbindungen **D** gelten insbesondere Verbindungen der Formel (XII)

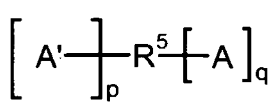 (XII)

wobei A' für eine Reaktivgruppe steht, welche ausgewählt ist aus der Gruppe umfassend

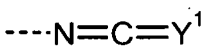 , 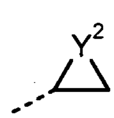 , 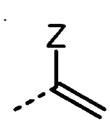 ,

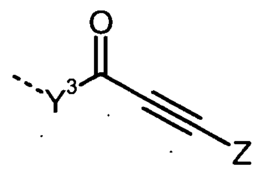 , 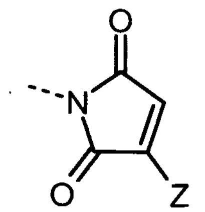 und 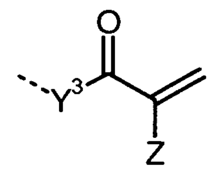

und wobei A' identisch mit der Reaktivgruppe A oder davon verschieden sein kann und die anderen Substituenten und Indizes wie für die Formel (I) beschrieben sind.

Beispiele für geeignete Verbindungen **D** sind

- zwei- oder mehrwertige, mono- und/oder oligomere aliphatische, cycloaliphatische, arylaliphatische und aromatische Isocyanate (Polyisocyanate) wie 1,6-Hexamethylendiisocyanat (HDI), 2-Methylpentamethylen-1,5-diisocyanat, 2,2,4- und 2,4,4-Trimethyl-1,6-hexamethylendiisocyanat (TMDI), 1,12-Dodecamethylendiisocyanat, Lysin- und Lysinesterdiisocyanat, Cyclohexan-1,3- und -1,4-diisocyanat und beliebige Gemische dieser Isomeren, 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), 1,3,5-Tris-(isocyanatomethyl)-benzol, m- und p-Tetramethyl-1,3- und -1,4-xylylendiisocyanat (m- und p-TMXDI), Bis-(1-Isocyanato-1-methylethyl)-naphthalin, α,α,α',α',α",α"-Hexamethyl-1 ,3,5-mesitylentriisocyanat, Dimer- und Trimerfettsäureisocyanate wie 3,6-Bis-(9-isocyanatononyl)-4,5-di-(1-heptenyl)-cyclohexen (Dimeryldiisocyanat), 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-düsocyanatodiphenyl (TODI), Tris-(4-isocyanatophenyl)-methan, Tris-(4-isocyanatophenyl)-thiophosphat; Oligomere dieser Isocyanate enthaltend Uretdion-, Isocyanurat- oder Iminooxadiazindiongruppen; modifizierte zwei- und mehrwertige Isocyanate enthaltend Ester-, Harnstoff-, Urethan-, Biuret-, Allophanat-, Carbodiimid-, Uretonimin- oder Oxadiazintriongruppen; sowie Isocyanat-haltige Polyurethanpolymere, das heisst mehr als eine Isocyanatgruppe aufweisende Umsetzungsprodukte von Polyisocyanaten mit zwei- oder mehr Hydroxylgruppen aufweisenden Substanzen (so genannten "Polyolen"), wie beispielsweise zwei- oder mehrwertigen Alkoholen, Glykolen oder Aminoalkoholen, polyhydroxyfunktionellen Polyethern, Polyestern, Polyacrylaten, Polycarbonaten oder Polykohlenwasserstoffen, insbesondere Polyethern;
- zwei- oder mehrwertige Epoxide (Polyepoxide) wie Bis-(2,3-epoxycyclopentyl)ether, Polyglycidylether von mehrwertigen aliphatischen und cycloaliphatischen Alkoholen wie 1,4-Butandiol, Polypropylenglykolen und 2,2-Bis-(4-hydroxycyclohexyl)-propan; Polyglycidylether von mehrwertigen Phenolen wie Resorcinol, Bis-(4-hydroxyphenyl)-methan (Bisphenol-F), 2,2-Bis-(4-hydroxyphenyl)-propan (Bisphenol-A), 2,2-Bis-(4-hydroxy-3,5-dibromophenyl)-propan, 1,1,2,2-Tetrakis-(4-hydroxyphenyl)-ethan, Kondensationsprodukten von Phenolen mit Formaldehyd, die unter sauren Bedingungen erhalten werden, wie Phenolnovolaken und Kresolnovolaken, sowie mit diesen Alkoholen und Phenolen, oder mit Polycarbonsäuren wie beispielsweise dimeren Fettsäuren, oder einem Gemisch davon, vorverlängerte Polyglycidylether; Polyglycidylester von mehrwertigen Carbonsäuren wie Phthalsäure, Terephthalsäure, Tetrahydrophthalsäure und Hexahydrophthalsäure; N-Glycidyl-Derivate von Aminen, Amiden und heterocyclischen Stickstoffbasen wie N,N-Diglycidylanilin, N,N-Diglycidyltoluidin, N,N,O-Triglycidyl-4-aminophenol, N,N,N',N'-Tetraglycidyl-bis-(4-aminophenyl)-methan, Triglycidylcyanurat und Triglycidylisocyanurat;

- zwei- oder mehrwertige, Acryl-, Methacryl- oder Acrylamidgruppen tragende Verbindungen wie Tris-(2-hydroxyethyl)-isocyanurat-tri(meth)acrylat, Tris-(2-hydroxyethyl)-cyanurat-tri(meth)acrylat, N,N',N"-Tris-(meth)acryloyl-perhydrotriazin; zwei- oder mehrwertige Acrylate und Methacrylate von aliphatischen Polyethern, Polyestern, Novolaken, Phenolen, aliphatischen oder cycloaliphatischen Alkoholen, Glykolen und Polyesterglykolen sowie mono- und polyalkoxylierten Derivaten der vorgenannten Verbindungen, beispielsweise Ethylenglykol-di(meth)acrylat, Tetraethylenglykol-di(meth)acrylat, Tripropylenglykol-di(meth)acrylat, Polyethylenglykol-di(meth)acrylat, Polypropylenglykol-di-(meth)acrylat, 1,4-Butandiol-di(meth)acrylat, 1,6-Hexandiol-di(meth)acrylat, Neopentylglykol-di(meth)acrylat, Trimethylolpropan-tri(meth)acrylat, Pentaerithritol-tetra(meth)acrylat, Dipentaerithritol-tetra(meth)acrylat, Dipentaerithritolpenta(meth)acrylat, Dipentaerithritol-hexa(meth)acrylat; zwei- oder mehrwertige Acryl- oder Methacryl-funktionelle Polybutadiene, Polyisoprene oder Blockcopolymere davon; Addukte aus zwei- oder mehrwertigen Epoxiden wie die oben genannten Epoxide mit Acryl- und Methacrylsäure; zwei- oder mehrwertige Polyurethan(meth)acrylate; zwei- oder mehrwertige Acrylamide wie N,N'-Methylen-bis-acrylamid;

- zwei- oder mehrwertige, 1-Ethinyicarbonyl- oder 1-Propinylcarbonylgruppen tragende Verbindungen;
- zwei- oder mehrwertige, Maleimid- oder Citraconimidgruppen tragende Verbindungen wie die Bis- und Polykismaleimide aus aliphatischen, cycloaliphatischen oder aromatischen Di- und Polyaminen und Malein- oder Citraconsäureanhydrid, beispielsweise $\alpha,\omega$-Dimerfettsäure-bis(maleimid), 4,4'-Diphenylmethan-bis(maleimid), 1,3-Xylylen-bis(citraconimid); Bis- und Polykismaleimide aus aminoterminierten Butadien/Acrylonitril-Copolymeren (beispielsweise erhältlich unter dem Namen Hycar® ATBN von Noveon) und Malein- oder Citraconsäureanhydrid; zwei- oder mehrwertige Addukte aus Di- und Polyisocyanaten mit N-Hydroxyethylmaleimid; Ester aus zwei- oder mehrwertigen Alkoholen und 6-Maleimidohexansäure;
- zwei- oder mehrwertige, Vinyl- und/oder Isopropenylgruppen tragende Verbindungen wie 1,3- und 1,4-Divinylbenzol, Divinylsulfon,Vinylcrotonat,Diallylidenpentaerythritol-acetal, 1,3-Diisopropenylbenzol und 1,3,5-Triisopropenylbenzol, 3-(2-Vinyloxyethoxy)-styrol, Divinyldimethylsilan, Trivinylmethylsilan, Trivinylmethoxysilan, Divinyltetramethyldisiloxan, 1,3-Divinyl-1,3-diphenyl-1,3-dimethyldisiloxan, 1,3-Divinyl-tetraethoxydisiloxan, Trivinyl-pentamethyltrisiloxan, 4-Vinyloxybutoxy-trivinylsilan, Tris-(4-vinyloxybutoxy)-vinylsilan; zwei- oder mehrwertiges Vinyl- und Isopropenylether wie Divinylether, Isopropenylvinylether, Triethylenglykol-divinylether, Butandiol-divinylether, Hexandiol-divinylether, Octadecandiol-divinylether, Dimerfettsäurediol-divinylether und Divinylbutyral; Divinylester von Dicarbonsäuren, beispielsweise Adipinsäuredivinylester;
- zwei- oder mehrwertige, Allylgruppen tragende Verbindungen wie Triallylcyanurat, Triallylisocyanurat, Triallylphosphat; zwei- oder mehrwertige Allylether von Alkoholen und Glykolen sowie mono- und polyalkoxylierten Derivaten davon; beispielsweise 1,4-Bis-(allyloxy)-butan, 1,6-Bis-(allyloxy)-hexan, Triethylenglykol-diallylether, Bisphenol-A-diallylether, 3,3'-Diallyl-bisphenol-A-diallylether, 3,3'-Diallyl-bisphenol-A, Trimethylolpropan-diallylether, Glycerin-triallylether, Trimethylolpropan-triallylether, Pentaerithritol-tetraallylether; zwei- oder mehrwertige Allylester und -amide von Carbonsäuren, beispielsweise Diallylphthalat, Diallyliso- und -terephthalat, Diallyloxalat, Diallylsebacat, Diallylmaleinat, Diallylfumarat, Diallylitaconat; zweiwertige Allylcarbonate wie Diallylcarbonat, Di- und Triethylengiykoi-bis-allylcarbonat; zwei- oder mehrwertige Addukte aus Di- und Polyisocyanaten mit Glycidol, Allylalkohol oder Allylglykolen, beispielsweise 1,6-Hexamethylen-bis-allylcarbamat;
- sowie zwei- oder mehrwertige heterofunktionelle, das heisst mindestens zwei verschiedene der vorgenannten Reaktivgruppen tragende, Verbindungen (A verschieden von A') wie 4-Allyloxy-phenylisocyanat, 1-Alkenylisocyanate wie Vinylisocyanat, Propenylisocyanat und Isopropenylisocyanat, 2-Isocyanatoethyl-methacrylat, 1,2-Dimethyl-3-isocyanatopropyl-acrylat, p-Isocyanatostyrol, m- und p-Isopropenyl-$\alpha,\alpha$-dimethylbenzylisocyanat (m- und p-TMI), m- und p-Ethenyl-$\alpha,\alpha$-dimethylbenzylisocyanat, Isopropenyl-$\alpha,\alpha,\alpha',\alpha'$-tetramethyl-xylylendiisocyanat, Glycidylallylether, Glycidoxy-trivinylsilan, Triglycidoxy-vinylsilan, N-(Trivinylsilyloxymethyl)-maleimid; heterofunktionelle Addukte aus Di- und Polyisocyanaten mit Glycidol, Allylalkohol, Allylglykolen, N-Hydroxyethylmaleimid, hydroxyfunktionellen Acrylat- und Methacrylaten wie 2-Hydroxyethyl-acrylat und -methacrylat; heterofunktionelle Addukte aus Mono- und Polycarbodiimiden von Di- und Polyisocyanaten mit Acryl- oder Methacrylsäure; heterofunktionelle Addukte aus zwei- oder mehrwertigen Epoxiden mit Acryl- oder Methacrylsäure, Vinylallylether, Ethylenglykolvinylallylether, Vinylallylphthalat, Ethylenglykol-(2-allylphenyl)-vinyl-ether, Allyl(meth)acrylat, Vinylacrylat, 2-Vinyloxyethyl-(meth)acrylat.

Als Verbindungen **D** insbesondere geeignet sind zwei- oder mehrwertige aliphatische; cycloaliphatische, arylaliphatische und aromatische Isocyanate, wie die erwähnten monomeren und oligomeren Polyisocyanate sowie die mehr als eine Isocyanatgruppe aufweisenden Umsetzungsprodukte von Polyisocyanaten mit Polyolen, insbesondere Polyetherpolyolen, Polyesterpolyolen, Polyacrylatpolyolen, Polycarbonatpolyolen, Polykohlenwasserstoffpolyolen und Mischungen dieser Polyole.

Je nach den Reaktivgruppen der Verbindung **D** und der den aktiven Wasserstoff tragenden Gruppe des Aldimins der Formel (XI) kann die Additionsreaktion nucleophil oder radikalisch erfolgen. Aus Gründen der Einfachheit soll der Begriff "Additionsreaktion" im vorliegenden Dokument auch ringöffnende Substitutionsreaktionen, wie sie beispielsweise Epoxide mit Nucleophilen eingehen, umfassen, weil das Ergebnis einer solchen, das Nucleofug nicht ais separates Molekül freisetzenden, Substitutionsreaktion dem einer Additionsreaktion gleichkommt. Die Additionsreaktion verläuft nucleophil, wenn die den aktiven Wasserstoff tragende Reaktivgruppe des Aldimins als Nucleophil an eine elektrophile Reaktivgruppe der Verbindung **D** angreift, beispielsweise im Fall des Angriffs einer Amino- oder Hydroxylgruppe an eine Isocyanatgruppe. Als Beispiel für eine radikalische Additionsreaktion kann die Reaktion einer Mercaptogruppe an eine Acrylgruppe genannt werden, wobei für diese Art der Additionsreaktion im Allgemeinen ein radikalbildender Initiator benötigt wird.

Die Umsetzung zwischen dem Aldimin der Formel (XI) und der Verbindung **D** zum Additionsprodukt, das heisst einer Aldimin-haltigen Verbindung der Formel (I), erfolgt unter bekannten Bedingungen, wie sie für Reaktionen zwischen den an der jeweiligen Umsetzung beteiligten Reaktivgruppen typischerweise verwendet werden, beispielsweise bei 20 bis 100˚C. Die Umsetzung erfolgt unter Verwendung eines Lösemittels oder bevorzugt lösemittelfrei. Gegebenenfalls können Hilfsstoffe wie beispielsweise Katalysatoren, Initiatoren oder Stabilisatoren mitverwendet werden. Die Umsetzung mit Isocyanaten wird für Aminoaldimine bevorzugt bei Raumtemperatur und ohne Katalysator, für Hydroxy-, Mercapto- und Harnstoffaldimine bei 40 bis 100˚C und unter Verwendung eines Katalysators, wie er für die Urethanisierungsreaktion zwischen Isocyanaten und Alkoholen verwendet wird, beispielsweise einer Organozinn-Verbindung, eines Bismutkomplexes, einer tertiären Aminverbindung oder einer Kombination solcher Katalysatoren, durchgeführt.

Wird die Reaktion stöchiometrisch geführt, das heisst mit einem Molequivalent aktiven Wasserstoff des Aldimins auf ein Molequivalent Reaktivgruppen der Verbindung **D -** wodurch deren Reaktivgruppen vollständig umgesetzt werden -, wird als Additionsprodukt ein Polyaldimin erhalten. Auf einfache Weise werden so vielfältige Polyaldimine erhalten, ohne dass zu ihrer Herstellung auf die entsprechenden primären Polyamine, welche technisch und kommerziell nur beschränkt zur Verfügung stehen, zurückgegriffen werden müsste. In Abhängigkeit von Struktur, Funktionalität und Molekulargewicht der Verbindungen **D** und der Aldimine der Formel (XI) können diese Polyaldimine höchst unterschiedliche Eigenschaften aufweisen; sie lassen sich also auf die Bedürfnisse einer bestimmten Anwendung massschneidern.

Durch geeignete Umsetzung der Aldimine der Formel (XI) mit Verbindungen **D** können auch Aldimin-haltige Verbindungen der Formel (I) hergestellt werden, welche heterofunktionell sind, das heisst, welche neben einer oder mehreren Aldiminogruppen noch eine oder mehrere andere, für Polyreaktionen zugängliche Reaktivgru,ppen aufweisen. Heterofunktionelle Aldimin-haltige Verbindungen der Formel (I) werden dann erhalten, wenn die Reaktion zwischen dem Aldimin der Formel (XI) und einer Verbindung **D** unterstöchiometrisch, das heisst mit weniger als einem Molequivalent aktiven Wasserstoff des Aldimins auf ein Molequivalent Reaktivgruppen der Verbindung **D,** geführt wird. Die Verbindung D selbst kann dabei homo- oder heterofunktionell sein.

Die auf die beschriebene Weise erhaltenen Additionsprodukte von Aldiminen der Formel (XI) mit Verbindungen **D,** d.h. Aldimin-haltige Verbindungen der Formel (I), sind, wie die Aldimine der Formel (XI), geruchsfrei. Sie sind unter geeigneten Bedingungen, insbesondere unter Ausschluss von Feuchtigkeit, lagerstabil. Heterofunktionelle Additionsprodukte, die neben Aldiminogruppen zusätzliche, für Polyreaktionen zugängliche Reaktivgruppen enthalten, sind dann lagerstabil, wenn sie überdies von Reaktionen dieser Reaktivgruppen auslösenden Faktoren, wie beispielsweise Hitze oder UV-Strahlung, ferngehalten werden.

Die Aldimine der Formel (XI) sowie die Aldimin-haltigen Verbindungen der Formel (I) lassen sich sehr breit verwenden. Grundsätzlich lassen sie sich überall dort einsetzen, wo sie als Quelle der Aldehyde der Formel (IV) und/oder der Amine **B** dienen können. Insbesondere können sie in der Funktion von geschützten Aminen, beziehungsweise geschützten Aldehyden, in Aldehyd- und/oder Amin-reaktiven Systemen eingesetzt und dort bei Bedarf gezielt entschützt werden. Insbesondere finden sie in Systemen Anwendungen, in welchen Verbindungen vorhanden sind, welche mit primären Aminen reagieren. Die Entschützung erfolgt hydrolytisch, beispielsweise durch Kontakt mit Wasser oder Feuchtigkeit, insbesondere Luftfeuchtigkeit.

Bei Zutritt von Feuchtigkeit können die Aldiminogruppen der Aldime der Formel (XI), sowie der Aldimin-haltigen Verbindungen der Formel (I) über Zwischenstufe formal zu Aminogruppen hydrolysieren, wobei der entsprechende, zur Herstellung des Aldimins der Formel (XI) verwendete Aldehyd **A** freigesetzt wird. Da diese Hydrolysereaktion reversibel ist und das chemische Gleichgewicht deutlich auf der Aldiminseite liegt, ist davon auszugehen, dass in Abwesenheit von gegenüber Aminen reaktiven Gruppen nur ein Teil der Aldiminogruppen teilweise oder vollständig hydrolysieren. Im speziellen Fall von heterofunktionellen Additionsprodukten, die gegenüber Aminen reaktive Gruppen, insbesondere Isocyanatgruppen, enthalten, reagieren die hydrolysierenden Aldiminogruppen hingegen weiter, beispielsweise mit Isocyanatgruppen zu Harnstoffgruppen. In diesen Fällen, wo in Formel (I) die Werte von m, p und q so gewählt sind, dass $m*p \leq q$ ist, kommt es zur Vernetzung des heterofunktionellen Additionsprodukts, welche auch ohne Beteiligung weiterer Substanzen direkt zu einem hochmolekularen Kunststoff führen kann. Die Reaktion der gegenüber Aminen reaktiven Gruppen mit den hydrolysierenden Aldiminogruppen muss dabei nicht notwendigerweise über Aminogruppen erfolgen. Selbstverständlich sind auch Reaktionen mit Zwischenstufen der Hydrolysereaktion möglich. Beispielsweise ist es denkbar, dass die hydrolysierende Aldiminogruppe in der Form eines Halbaminals direkt mit den gegenüber Aminen reaktiven Gruppen reagiert.

Als Katalysatoren für die Hydrolyse der Aldiminogruppen eignen sich beispielsweise organische Carbonsäuren wie Benzoesäure, Salicylsäure oder 2-Nitrobenzoesäure, organische Carbonsäureanhydride wie Phthalsäureanhydrid oder Hexahydrophthalsäureanhydrid, Silylester von organischen Carbonsäuren, organische Sulfonsäuren wie Methansulfonsäure, p-Toluolsulfonsäure oder 4-Dodecylbenzolsulfonsäure, oder andere organische oder anorganische Säuren, oder Mischungen der vorgenannten Säuren.

Die Aldimin-haltigen Verbindungen der Formel (I) lassen sich beispielsweise als Bausteine für Kunststoffvorläufer verwenden. Mit dem Begriff "Kunststoffvorläufer" werden im vorliegenden Dokument monomere, oligomere oder polymere organische Verbindungen - oder solche Verbindungen zu einem wesentlichen Teil enthaltende homogene oder heterogene Zusammensetzungen - bezeichnet, welche aufgrund von in ihnen enthaltenen, für Polyreaktionen zugänglichen Reaktivgruppen befähigt sind, mit sich allein oder zusammen mit anderen Molekülen zu hochmolekularen Kunststoffen, das heisst organischen Polymeren, auszureagieren, ein Prozess, der gemeinhin als "Aushärtung" oder auch als "Vernetzung" bezeichnet wird - unabhängig davon, ob die bei der Aushärtung ablaufenden Reaktionen zu kovalent oder andersartig vernetzten Strukturen führen. Der Begriff "Polyreaktionen" umfasst dabei sämtliche Arten von

Polyadditions-, Polykondensation- und Polymerisationsreaktionen. Der Begriff "Polymer" umfasst im vorliegenden Dokument sowohl ein Kollektiv von chemisch einheitlichen, sich aber in Bezug auf Polymerisationsgrad, Molmasse und Kettenlänge unterscheidenden Makromolekülen, die durch eine Polyreaktion hergestellt wurden, als auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Die Vorsilbe "Poly" in Substanzbezeichnungen, wie "Polyaldimin", "Polyamin", "Polyisocyanat" oder "Polyol" weist im vorliegenden Dokument darauf hin, dass die jeweilige Substanz formal mehr als eine der in ihrer Bezeichnung vorkommenden funktionellen Gruppe pro Molekül enthält.

Rein aldiminofunktionelle, Polyaldimine darstellende Verbindungen der Formel (I) lassen sich als Bausteine, insbesondere als latente Härter, für Kunststoffvorläufer verwenden, welche Reaktivgruppen enthalten, die gegenüber Aldiminogruppen als solchen oder hydrolysierten Formen davon reaktiv sind. Heterofunktionelle Verbindungen der Formel (I), welche zusätzlich zu den Aldiminogruppen noch mindestens eine weitere, für Polyreaktionen zugängliche Reaktivgruppe enthalten, sind verwendbar als Bausteine, beispielsweise als Co-Monomere oder latente Härter, für Kunststoffvorläufer; oder, wenn die Aldiminogruppe als solche oder nach ihrer Hydrolyse mit den anderen in der Verbindung der Formel (I) vorliegenden Reaktivgruppen unter Verknüpfung der Moleküle reagieren kann, auch als Kunststoffvorläufer selbst. Dies trifft insbesondere zu für den Fall von Aldimin-haltigen Verbindungen der Formel (I), die zusätzlich Isocyanatgruppen enthalten.

Geeignete Kunststoffvorläufer, in welchen sich die beschriebenen Aldimin-haltigen Verbindungen der Formel (I) als Bausteine, beispielsweise als latente Härter oder als Co-Monomere, einsetzen lassen, sind solche, welche Substanzen mit mindestens einem Typ von Reaktivgruppen enthalten, die mit Aldiminen als solchen oder nach deren teilweisen oder vollständigen Hydrolyse Reaktionen eingehen, die für sich allein oder in Kombination mit weiteren Reaktionen zur Vernetzung des Kunststoffvorläufers führen. Beispiele für solche Reaktivgruppen sind Isocyanat-, Isothiocyanat-, Epoxid-, Episulfid- und Cyclocarbonatgruppen. Die Reaktionen dieser Reaktivgruppen mit den Aldiminogruppen können durch Feuchtigkeit und/oder durch Hitze ausgelöst werden. Insbesondere geeignete Reaktivgruppen sind Isocyanatgruppen, Isothiocyanatgruppen und Epoxidgruppen. Als Kunststoffvorläufer geeignet sind auch solche Kunststoffvorläufer, welche neben den Substanzen mit den genannten Reaktivgruppen weitere Substanzen mit für Polyreaktionen zugänglichen Gruppen, wie beispielsweise Aziridin-, Acryl-, Methacryl-, 1-Ethinylcarbonyl-, 1-Propinylcarbonyl-, Maleimid-, Citraconimid-, Vinyl-, Isopropenyl-, Allyl- oder Silanolgruppen, oder zu Silanolgruppen hydrolysierende Gruppen, enthalten.

Geeignete Kunststoffvorläufer, für welche sich die Aldimin-haltigen Verbindungen der Formel (I) als Bausteine, beispielsweise als latente Härter oder als Co-Monomere, verwenden lassen, sind solche, welche mindestens einen Typ der vorgenannten Reaktivgruppen enthalten, insbesondere Isocyanatgruppen, Isothiocyanatgruppen und Epoxidgruppen. Als Kunststoffvorläufer geeignet sind auch heterofunktionelle Kunststoffvorläufer, das heisst Kunststoffvorläufer, welche zwei oder mehr verschiedene Typen von für Polyreaktionen zugänglichen Reaktivgruppen wie Isocyanat-, Isothiocyanat-, Epoxid-, Episulfid-, Cyclocarbonat-, Aziridin-, Acryl-, Methacryl-, 1-Ethinylcarbonyl-, 1-Propinylcarbonyl-, Maleimid-, Citraconimid-, Vinyl-, Isopropenyl-, Allyl- oder Silanolgruppen, oder zu Silanolgruppen hydrolysierende Gruppen, enthalten.

Insbesondere geeignete Kunststoffvorläufer, in welchen sich die Aldimin-haltigen Verbindunge der Formel (I) einsetzen lassen, sind Isocyanat-haltige Zusammensetzungen, das heisst solche Kunststoffvorläufer, welche als Reaktivgruppen ausschliesslich oder zu einem wesentlichen Teil Isocyanatgruppen enthalten, die Bestandteil von Polyurethanpolymeren und/oder von Polyisocyanaten sind.

Geeignete Kunststoffvorläufer, welche die Aldimin-haltige Verbindungen der Formel (i) ais Bestandteile enthalten, können einkomponentige oder zweikomponentig sein.

Als einkomponentige Kunststoffvorläufer insbesondere geeignet sind einkomponentige Isocyanat-haltige Zusammensetzungen, welche mindestens eine Aldimin-haltige Verbindung der Formel (I) und mindestens ein Isocyanat-haltiges Polyurethanpolymer **P,** das ein Umsetzungsprodukt aus Polyisocyanaten und Polyolen darstellt, enthalten. Der Begriff "Polyurethanpolymer" umfasst im vorliegenden Dokument sämtliche Polymere, welche nach dem Diisocyanat-Polyadditions-Verfahren hergestellt werden. Dies schliesst auch solche Polymere ein, die nahezu oder gänzlich frei sind von Urethangruppen, wie Polyether-Polyurethane, Polyester-Polyurethane, Polyether-Polyharnstoffe, Polyharnstoffe, Polyester-Polyharnstoffe, Polyisocyanurate, Polycarbodiimide, usw.

Das Isocyanat-haltige Polyurethanpolymer **P** wird hergestellt durch Umsetzung von mindestens einem Polyol mit mindestens einem Polyisocyanat. Diese Umsetzung kann dadurch erfolgen, dass das Polyol und das Polyisocyanat mit üblichen Verfahren, beispielsweise bei Temperaturen von 50˚C bis 100˚ C, gegebenenfalls unter Mitverwendung geeigneter Katalysatoren, zur Reaktion gebracht werden, wobei das Polyisocyanat so dosiert ist, dass dessen Isocyanatgruppen im Verhältnis zu den Hydroxylgruppen des Polyols im stöchiometrischen Überschuss vorhanden sind. Der Überschuss an Polyisocyanat wird so gewählt, dass im resultierenden Polyurethanpolymer **P** nach der Umsetzung aller Hydroxylgruppen des Polyols beispielsweise ein Gehalt an freien Isocyanatgruppen von 0.1 - 15 Gewichts-%, insbesondere 0.5 - 5 Gewichts-%, bezogen auf das gesamte Polyurethanpolymer **P,** verbleibt. Gegebenenfalls kann das

Polyurethanpolymer **P** unter Mitverwendung von Weichmachern hergestellt werden, wobei die verwendeten Weichmacher keine gegenüber Isocyanaten reaktiven Gruppen enthalten.

Als Polyole für die Herstellung eines solchen Isocyanat-haltigen Polyurethanpolymers **P** können beispielsweise die folgenden, handelsüblichen Polyole, oder beliebige Mischungen davon, eingesetzt werden:

- Polyoxyalkylenpolyole, auch Polyetherpolyole oder Oligoetherole genannt, welche Polymerisationsprodukte von Ethylenoxid, 1,2-Propylenoxid, 1,2- oder 2,3-Butylenoxid, Tetrahydrofuran oder Mischungen davon sind, eventuell polymerisiert mit Hilfe eines Startermoleküls mit zwei oder mehreren aktiven Wasserstoffatomen wie beispielsweise Wasser, Ammoniak oder Verbindungen mit mehreren OH- oder NH-Gruppen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.

Besonders geeignet sind Polyoxyalkylendiole oder Polyoxyalkylentriole, insbesondere Polyoxypropylendiole oder Polyoxypropylentriole.

Speziell geeignet sind Polyoxyalkylendiole oder Polyoxyalkylentriole mit einem Ungesättigtheitsgrad tiefer als 0.02 mEq/g und mit einem Molekulargewicht im Bereich von 1'000 - 30'000 g/mol, sowie Polyoxypropylendiole und -triole mit einem Molekulargewicht von 400 - 8'000 g/mol. Der Begriff "Molekulargewicht" bezeichnet im vorliegenden Dokument das Molekulargewichtsmittel $M_n$. Ebenfalls besonders geeignet sind sogenannte Ethylenoxid-terminierte ("EO-endcapped", ethylene oxide-endcapped) Polyoxypropylenpolyole. Letztere sind spezielle Polyoxypropylenpolyoxyethylenpolyole, die beispielsweise dadurch erhalten werden, dass reine Polyoxypropylenpolyole, insbesondere Polyoxypropylendiole und -triole, nach Abschluss der Polypropoxylierungsreaktion mit Ethylenoxid weiter alkoxyliert werden und dadurch primäre Hydroxylgruppen aufweisen.

- Styrol-Acrylnitril- oder Acrylnitril-Methylmethacrylat-gepfropfte Polyetherpolyole.
- Polyesterpolyole, auch Oligoesterole genannt, hergestellt beispielsweise aus zwei- bis dreiwertigen Alkoholen wie beispielsweise 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1-Trimethylolpropan oder Mischungen der vorgenannten Alkohole mit organischen Dicarbonsäuren oder deren Anhydride oder Ester wie beispielsweise Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure und Hexahydrophthalsäure oder Mischungen der vorgenannten Säuren, sowie Polyesterpolyole aus Lactonen wie beispielsweise ε-Caprolacton.

- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Polyacrylat- und Polymethacrylatpolyole.
- Polykohlenwasserstoffpolyole, auch Oligohydrocarbonole genannt, wie beispielsweise polyhydroxyfunktionelle Ethylen-Propylen-, Ethylen-Butylen- oder Ethylen-Propylen-Dien-Copolymere, wie sie beispielsweise von der Firma Kraton Polymers hergestellt werden, oder polyhydroxyfunktionelle Copolymere aus Dienen wie 1,3-Butandien oder Diengemischen und Vinylmonomeren wie Styrol, Acrylnitril oder Isobutylen, oder polyhydroxyfunktionelle Polybutadienpolyole, wie beispielsweise solche, die durch Copolymerisation von 1,3-Butadien und Allylalkohol hergestellt werden.
- Polyhydroxyfunktionelle Acrylonitril/Polybutadien-Copolymere, wie sie beispielsweise aus Epoxiden oder Aminoalkoholen und carboxylterminierten Acrylonitril/Polybutadien-Copolymeren (kommerziell erhältlich unter dem Namen Hycar® CTBN von Hanse Chemie) hergestellt werden können.

Diese genannten Polyole weisen ein mittleres Molekulargewicht von 250 - 30'000 g/mol, insbesondere von 1'000 - 30'000 g/mol, und eine mittlere OH-Funktionalität im Bereich von 1.6 bis 3 auf.

Zusätzlich zu diesen genannten Polyolen können kleine Mengen von niedrigmolekularen zwei- oder mehrwertigen Alkoholen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, hydriertes Bisphenol A, dimere Fettalkohole, 1,1,1-Trimethylolethan, 1,1,1-Trimethylolpropan, Glycerin, Pentaerythrit, Zuckeralkohole wie Xylit, Sorbit oder Mannit, Zucker wie Saccharose, andere höherwertige Alkohole, niedrigmolekulare Alkoxylierungsprodukte der vorgenannten zwei- und mehrwertigen Alkohole, sowie Mischungen der vorgenannten Alkohole bei der Herstellung des Polyurethanpolymers **P** mitverwendet werden.

Als Polyisocyanate für die Herstellung eines solchen Isocyanat-haltigen Polyurethanpolymers werden die mono- oder oligomeren zwei- oder mehrwertigen Isocyanate verwendet, wie sie als Verbindungen **D** geeignet genannt wurden. Als Polyisocyanate insbesondere geeignet sind MDI, HDI, TDI und IPDI.

Der einkomponentige Kunststoffvorläufer enthält mindestens eine Aldimin-haltige Verbindung der Formel (I), insbesondere in einer der oben bereits im Detail beschriebenen bevorzugten Ausführungsform. Die Aldimin-haltige Verbindung der Formel (I) kann dabei gesondert hergestellt und als solche in den Kunststoffvorläufer eingearbeitet werden. Sie kann aber auch in situ, das heisst im Zuge der Herstellung des Kunststoffvorläufers, hergestellt werden, indem geeignete Mengen von mindestens einem Aldimin der Formel (XI) und mindestens einer Verbindung **D** in situ, das heisst in Anwesenheit weiterer Bestandteile des Kunststoffvorläufers, zum Additionsprodukt umgesetzt werden. Insbesondere kann zur Herstellung der beschriebenen einkomponentigen Isocyanat-haltigen Zusammensetzungen so vorgegangen werden, dass geeignete Mengen von mindestens einem Aldimin der Formel (XI) und mindestens einer Verbindung **D** in situ umgesetzt werden, wobei die Verbindung **D** bevorzugt ein Isocyanat-haltiges Polyurethanpolymer darstellt, wie es oben als Polyurethanpolymer **P** im Detail beschrieben wurde.

Die einkomponentige Isocyanat-haltige Zusammensetzung enthält mindestens eine Aldimin-haltige Verbindung der Formel (I), beziehungsweise eine bevorzugte Ausführungsform davon, wie es, beziehungsweise sie, oben bereits im Detail beschrieben worden sind. Die Aldimin-haltige Verbindung der Formel (I) kann dabei gesondert hergestellt und mit dem Isocyanat-haltigen Polyurethanpolymer **P** vermischt werden. Sie kann aber auch in situ, das heisst im Zuge der Herstellung der Isocyanat-haltigen Zusammensetzung, hergestellt werden, indem mindestens ein Aldimin der Formel (XI) mit mindestens einem Isocyanat-haltigen Polyurethanpolymer **P** in einem geeigneten stöchiometrischen Verhältnis miteinander vermischt und damit zum Additionsprodukt umgesetzt werden.

Typischerweise ist die Aldimin-haltige Verbindung der Formel (I) in einer Menge von 0.1 bis 30 Gewichts-%, bevorzugt 0.5 bis 20 Gewichts-%, und insbesondere 1 bis 10 Gewichts-%, bezogen auf den einkomponentigen Kunststoffvorläufer, insbesondere die einkomponentige Isocyanat-haltige Zusammensetzung, vorhanden.

Für den Fall, dass die Aldimin-haltige Verbindung der Formel (I) ein freie Isocyanatgruppen aufweisendes Umsetzungsprodukt aus einem Isocyanat-haltigen Polyurethanpolymer und einem Aldimin der Formel (XI) darstellt, kann der Gehalt der Aldimin-haltigen Verbindung im einkomponentigen Kunststoffvorläufer auch bis gegen 100 Gewichts-% betragen, da eine derartige Zusammensetzung unter dem Einfluss von Wasser vernetzt.

Es ist vorteilhaft, wenn die einkomponentige Isocyanat-haltige Zusammensetzung zusätzlich zur Aldimin-haltigen Verbindung der Formel (I) und zum Polyurethanpolymer **P** mindestens einen Katalysator **KAT-1** enthält. Als Katalysator **KAT-1** geeignet sind Verbindungen, welche zusammen mit Isocyanat-gruppen lagerstabil sind, und welche die zur Aushärtung der Zusammensetzung führenden Reaktionen der Isocyanatgruppen, insbesondere jene mit Aldiminogruppen sowie mit Feuchtigkeit, beschleunigen. Als geeignete Katalysatoren **KAT-1** genannt werden sollen Säuren, beispielsweise organische Carbonsäuren wie Benzoesäure, Salicylsäure oder 2-Nitrobenzoesäure, organische Carbonsäureanhydride wie Phthalsäureanhydrid oder Hexahydrophthalsäureanhydrid, Silylester von organischen Carbonsäuren, organische Sulfonsäuren wie Methansulfonsäure, p-Toluolsulfonsäure oder 4-Dodecylbenzolsulfonsäure, oder weitere organische oder anorganische Säuren; Metallverbindungen, beispielsweise Zinnverbindungen, zum Beispiel Dialkylzinndicarboxylate wie Dibutylzinndiacetat, Dibutylzinn-bis-(2-ethylhexanoat), Dibutylzinndilaurat, Dibutylzinndipalmitat, Dibutylzinndistearat, Dibutylzinndioleat, Dibutylzinndilinolat, Dibutylzinndilinolenat, Dibutylzinndiacetylacetonat, Dibutylzinnmaleat, Dibutylzinn-bis-(octylmaleinat), Dibutylzinnphthalat, Dimethylzinndilaurat, Dioctylzinndiacetat oder Dioctylzinndilaurat, Dialkylzinnmercaptide wie Dibutylzinn-bis-(2-ethylhexylmercaptoacetat) oder Dioctylzinn-bis-(2-ethylhexyl mercaptoacetat), Dibutylzinndichlorid, Monobutylzinntrichlorid, Alkylzinnthioester, Dibutylzinnoxid, Dioctylzinnoxid, Zinn(II)-carboxylate wie Zinn(II)-octoat, Zinn(II)-2-ethylhexanoat, Zinn(II)-laurat, Zinn(II)-oleat oder Zinn (II)-naphtenat, Stannoxane wie Laurylstannoxan, Bismutverbindungen wie Bismut(III)-octoat, Bismut(III)-neodecanoat oder Bismut(III)-oxinate; schwach basische tertiäre Aminverbindungen wie beispielsweise 2,2'-Dimorpholinodiethylether und andere Morpholinether-Derivate; sowie Kombinationen der genannten Verbindungen, insbesondere von Säuren und Metallverbindungen oder von Metallverbindungen und aminogruppenhaltigen Verbindungen.

Der einkomponentige Kunststoffvorläufer enthält gegebenenfalls weitere Bestandteile, wie sie nach dem Stand der Technik üblicherweise eingesetzt werden. Insbesondere enthält die einkomponentige Isocyanat-haltige Zusammensetzung gegebenenfalls eines oder mehrere der folgenden Hilfs- und Zusatzmittel:

- Weichmacher, beispielsweise Ester organischer Carbonsäuren oder deren Anhydride, Phthalate, wie beispielsweise Dioctylphthalat oder Diisodecylphthalat, Adipate, wie zum Beispiel Dioctyladipat, Sebacate, Polyole wie beispielsweise Polyoxyalkylenpolyole oder Polyesterpolyole, organische Phosphor- und Sulfonsäureester oder Polybutene;
- Lösemittel, beispielsweise Ketone wie Aceton, Methylethylketon, Diisobutylketon,Acetonylaceton,Mesityloxid,sowie

cyclische Ketone wie Methylcyclohexanon und Cyclohexanon; Ester wie Ethylacetat, Propylacetat oder Butylacetat, Formiate, Propionate oder Malonate; Ether wie Ketonether, Esterether und Dialkylether wie Diisopropylether, Diethylether, Dibutylether, Diethylenglykoldiethylether sowie Ethylenglykoldiethylether; aliphatische und aromatische Kohlenwasserstoffe wie Toluol, Xylol, Heptan, Octan sowie unterschiedliche Erdölfraktionen wie Naphtha, White Spirit, Petrolether oder Benzin;halogenierte Kohlenwasserstoffe wie Methylenchlorid; sowie N-alkylierte Lactame wie beispielsweise N-Methylpyrrolidon,N-Cyclohexylpyrrolidon oder N-Dodecylpyrrolidon;

- anorganische und organische Füllstoffe, wie zum Beispiel gemahlene oder gefällte Calciumcarbonate, welche gegebenenfalls mit Stearaten beschichtet sind, insbesondere feinteiliges beschichtetes Calciumcarbonat, Russe, Kaoline, Aluminiumoxide, Kieselsäuren, PVC-Pulver oder Hohlkugeln; Fasern, beispielsweise aus Polyethylen; Pigmente;
- weitere in der Polyurethanchemie übliche Katalysatoren;
- Reaktivverdünner und Vernetzer, beispielsweise Polyisocyanate wie MDI, PMDI, TDI, HDI, 1,12-Dodecamethylendiisocyanat, Cyclohexan-1,3- oder 1,4-diisocyanat, IPDI, Perhydro-2,4'- und -4,4'-diphenylmethandiisocyanat, 1,3- und 1,4-Tetramethylxylylendiisocyanat, Oligomere und Polymere dieser Polyisocyanate, insbesondere Isocyanurate, Carbodiimide, Uretonimine, Biurete, Allophanate und Iminooxadiazindione der genannten Polyisocyanate, Addukte von Polyisocyanaten mit kurzkettigen Polyolen, sowie Adipinsäuredihydrazid und andere Dihydrazide;
- latente Polyamine wie beispielsweise Polyaldimine, Polyketimine, Polyenamine, Polyoxazolidine, an einen Zeolith adsorbierte oder mikroverkapselte Polyamine sowie Amin-Metallkomplexe, bevorzugt Polyaldimine Polyaldimine aus der Umsetzung eines primären aliphatischen Polyamins mit einem Aldehyd, insbesondere einem Aldehyd **A** wie beispielsweise 2,2-Dimethyl-3-acyloxy-propanal, insbesondere 2,2-Dimethyl-3-lauroyloxy-propanal, sowie Komplexe zwischen Methylendianilin (MDA) und Natriumchlorid (erhältlich als Dispersion in Diethylhexylphthalat oder Diisodecylphthalat unter dem Handelsnamen Caytur® 21 von Crompton Chemical);
- Trocknungsmittel, wie beispielsweise p-Tosylisocyanat und andere reaktive Isocyanate, Orthoameisensäureester, Calciumoxid; Vinyltrimethoxysilan oder andere schnell hydrolysierende Silane wie beispielsweise Organoalkoxysilane, welche in $\alpha$-Stellung zur Silangruppe eine funktionelle Gruppe aufweisen, oder Molekularsiebe;
- Rheologie-Modifizierer wie beispielsweise Verdickungsmittel, zum Beispiel Harnstoffverbindungen, Polyamidwachse, Bentonite oder pyrogene Kieselsäuren;
- Haftvermittler,insbesondere Silane wie beispielsweise Epoxysilane,Vinylsilane,(Meth)acrylsilane, Isocyanatosilane, Carbamatosilane,S-(Alkylcarbonyl)-mercaptosilane und Aldiminosilane, sowie oligomere Formen dieser Silane;
- Stabilisatoren gegen Wärme, Licht- und UV-Strahlung; flammhemmende Substanzen;
- oberflächenaktive Substanzen wie beispielsweise Netzmittel, Verlaufsmittel, Entlüftungsmittel oder Entschäumer;
- Biozide wie beispielsweise Algizide, Fungizide oder das Pilzwachstum hemmende Substanzen;

sowie weitere üblicherweise in einkomponentigen Isocyanat-haltigen Zusammensetzungen eingesetzte Substanzen.

Der einkomponentige Kunststoffvorläufer, insbesondere die einkomponentige Isocyanat-haltige Zusammensetzung, verfügt in Abwesenheit von die Vernetzungsreaktionen der im Kunststoffvorläufer vorhandenen Reaktivgruppen auslösenden Faktoren, insbesondere von Feuchtigkeit, Hitze oder UV-Strahlung, über eine gute Lagerstabilität. Insbesondere verfügt die einkomponentige Isocyanat-haltige Zusammensetzung in Abwesenheit von Feuchtigkeit, beispielsweise in einer klimadichten Verpackung oder Anordnung, wie beispielsweise in einem Fass, einem Beutel oder einer Kartusche, über eine gute Lagerstabilität. Mit den Begriffen "lagerstabil" und "Lagerstabilität" in Zusammenhang mit einem Kunststoffvorläufer wird im vorliegenden Dokument der Sachverhalt bezeichnet, dass die Viskosität des Kunststoffvorläufers bei geeigneter Lagerung in der betrachteten Zeitspanne nicht oder höchstens so stark ansteigt, dass der Kunststoffvorläufer auf die vorgesehene Weise verwendbar bleibt.

Unter dem Einfluss von Feuchtigkeit, beispielsweise beim Kontakt mit feuchter Luft oder nach dem Zumischen von Wasser, oder bei starker Erwärmung, oder unter dem Einfluss von UV-Strahlung, oder unter dem Einfluss einer Kombination dieser Faktoren, härtet der Kunststoffvorläufer rasch zu einem hochmolekularen Kunststoff aus. Insbesondere härtet die Isocyanat-haltige Zusammensetzung unter dem Einfluss von Feuchtigkeit schnell und vollständig zu einem weitgehend klebefreien Polyurethankunststoff aus. Die Aushärtung verläuft ohne Blasenbildung, da die Isocyanatgruppen teilweise oder vollständig mit den hydrolysierenden Aldiminogruppen reagieren, wobei nur wenig oder gar kein $CO_2$ entsteht. Die Aushärtung wird durch die Anwesenheit von Katalysatoren für die Hydrolyse der Aldiminogruppen, beispielsweise die bereits genannten organischen Carbonsäuren oder Sulfonsäuren, zusätzlich beschleunigt, ohne dass es dabei zur Blasenbildung kommt. Die zur Aushärtung benötigte Feuchtigkeit kann aus der Luft stammen (Luftfeuchtigkeit), wobei der Kunststoffvorläufer durch die Diffusion der Feuchtigkeit von aussen nach innen aushärtet. Der Kunststoffvorläufer kann aber auch mit einer Wasser enthaltenden Komponente in Kontakt gebracht werden, zum Beispiel durch Bestreichen, beispielsweise mit einem Abglättmittel, durch Besprühen oder mittels Eintauchverfahren, oder es kann dem Kunststoffvorläufer eine Wasser enthaltende Komponente zugesetzt werden, zum Beispiel in Form einer wasserhaltigen Paste, die beispielsweise über einen Statikmischer homogen oder heterogen mit dem Kunststoffvorläufer vermischt wird.

Wie bereits erwähnt, können geeignete Kunststoffvorläufer, welche Aldimin-haltige Verbindungen der Formel (I) als Bestandteile enthalten, auch zweikomponentig sein. Geeignete zweikomponentige Kunststoffvorläufer bestehen aus zwei Komponenten **K1** und **K2,** wobei mindestens eine der Komponenten **K1** oder **K2** mindestens eine Aldimin-haltige Verbindung der Formel (I) enthält, und wobei die Mischung der beiden Komponenten **K1** und **K2** zu einem hochmolekularen Kunststoff führt.

Als zweikomponentige Kunststoffvorläufer insbesondere geeignet sind zweikomponentige Isocyanat-haltige Zusammensetzungen, in welchen die Komponente **K1** mindestens ein Polyisocyanat und/oder mindestens ein Isocyanat-haltiges Polyurethanpolymer **P** umfasst, und die Komponente **K2** mindestens ein Polyol und/oder mindestens ein Polyamin umfasst, und mindestens eine der Komponenten **K1** oder **K2** mindestens eine Aldimin-haltige Verbindung der Formel (I) enthält.

Als Polyisocyanat der Komponente **K1** geeignet sind die für die Herstellung des Isocyanat-haltigen Polyurethanpolymers **P** genannten Polyisocyanate. Insbesondere geeignet ist PMDI ("polymeres MDI"), bekannt beispielsweise unter Handelsnamen wie Desmodur® VL, Desmodur® VL 50, Desmodur® VL R 10, Desmodur® VL R 20, Desmodur® VKS 20 F (alle von Bayer), Isonate® M 309, Voranate® M 229, Voranate M® 580 (alle von Dow) oder Lupranat® M 10 R (von BASF), sowie bei Raumtemperatur flüssige Formen von MDI (sogenanntes "modifiziertes MDI"), welche Gemische von MDI mit MDI-Derivaten, wie beispielsweise MDI-Carbodiimiden oder MDI-Uretoniminen, darstellen, bekannt beispielsweise unter Handelsnamen wie Desmodur® CD, Desmodur® PF, Desmodur® PC (alle von Bayer).

Als Isocyanat-haltige Polyurethanpolymere **P** der Komponente K1 insbesondere geeignet sind solche, welche unter Verwendung von MDI, HDI, TDI oder IPDI hergestellt wurden.

Als Polyole der Komponente **K2** geeignet sind dieselben Polyole, welche bereits als geeignet für die Herstellung des Isocyanat-haltigen Polyurethanpolymers **P** genannt wurden. Insbesondere geeignet sind hochfunktionelle Polyole, beispielsweise Triole, Tetrole und höherfunktionelle Polyole; aminhaltige bzw. mit Aminen (beispielsweise Ethylendiamin) gestartete Polyetherpolyole; kurzkettige Polyetherpolyole mit Molekulargewichten von 300 bis 2000; hydrophobe Polyole, insbesondere Fettpolyole wie beispielsweise Ricinusöl oder die unter dem Handelsnamen Sovermol® von Cognis bekannten Polyole; ebenso Diol-Kettenverlängerer wie 1,4-Butandiol, 1,6-Hexandiol, Ethylenglykol, Diethylenglykol, Propylenglykol, Dipropylenglykol, 1,4-Bis-(hydroxyethyl)-hydrochinon, 1,4-Cyclohexandiol oder N,N'-Bis-(hydroxyethyl)-piperazin.

Als Polyamine der Komponente **K2** geeignet sind zum einen primäre aliphatische Polyamine, wie sie als Amine **C** beschrieben wurden; zum anderen Polyaminoamide; sekundäre aliphatische Polyamine wie beispielsweise N,N'-Dibutyl-ethylendiamin; N,N'-Di-tert.butyl-ethylendiamin, N,N'-Diethyl-1,6-hexandiamin, 1-(1-Methylethyl-amino)-3-(1-methylethyl-aminomethyl)-3,5,5-trimethylcyclohexan (Jefflink® 754 von Huntsman), N4-Cyclohexyl-2-methyl-N2-(2-methylpropyl)-2,4-pentandiamin, N,N'-Dialkyl-1,3-xylylendiamin, Bis-(4-(N-alkylamino)-cyclohexyl)-methan, N-alkylierte Polyetheramine, Produkte aus der Michael-artigen Addition der beispielhaft erwähnten primären aliphatischen Polyaminen an Michaelakzeptoren wie Maleinsäurediester, Fumarsäurediester, Citraconsäurediester, Acrylsäureester, Methacrylsäureester, Zimtsäureester, Itaconsäurediester, Vinylphosphonsäurediester, Vinylsulfonsäurearylester, Vinylsulfone, Vinylnitrile, 1-Nitroethylene oder Knoevenagel-Kondensationsprodukte wie beispielsweise solche aus Malonsäurediestern und Aldehyden wie Formaldehyd, Acetaldehyd oder Benzaldehyd; aliphatische Polyamine mit primären und sekundären Aminogruppen, wie beispielsweise N-Butyl-1,6-hexandiamin; sowie primäre und/oder sekundäre aromatische Polyamine wie beispielsweise m- und p-Phenylendiamin, 4,4'-Diaminodiphenylmethan (MDA), 3,3'-Dichloro-4,4'-diaminodiphenylmethan (MOCA), Mischungen von 3,5-Dimethylthio-2,4- und -2,6-toluylendiamin (erhältlich als Ethacure® 300 von Albemarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)-benzolsulfonamid, 5,5'-Methylendianthranilsäure, Dimethyl-(5,5'-Methylendianthranilat), 1,3-Propylen-bis-(4-aminobenzoat), 1,4-Butylen-bis-(4-aminobenzoat), Polytetramethylenoxid-bis-(4-aminobenzoat) (erhältlich als Versalink® von Air Products), 1,2-Bis-(2-aminophenylthio)-ethan, N,N'-Dialkyl-p-phenylendiamin, N,N'-Dialkyl-4,4'-diaminodiphenylmethan, 2-Methylpropyl-(4-chloro-3,5-diaminobenzoat) und tert.Butyl-(4-chloro-3,5-diaminobenzoat).

Es ist auch möglich, Polyamine in Form von Derivaten einzusetzen, in denen alle oder ein Teil der Aminogruppen blockiert sind und erst nach ihrer Aktivierung durch Hydrolyse und/oder Erwärmen mit Isocyanaten reagieren. Beispiele für solche Polyamin-Derivate mit blockierten Aminogruppen sind mehrfunktionelle Aldimine, Ketimine, Enamine, Oxazolidine, Aminale, Ammoniumcarbonate, Amin-Kohlensäure-Salze (Carbamate) oder Amin-Metallkomplexe. Ebenfalls können an Zeolith adsorbierte oder mikroverkapselte Polyamine eingesetzt werden.

Die zweikomponentige Isocyanat-haltige Zusammensetzung enthält mindestens eine Aldimin-haltige Verbindung der Formel (I) in einer der oben bereits im Detail beschriebenen bevorzugten Ausführungsform.

Typischerweise ist die Aldimin-haltige Verbindung der Formel (I) in einer Menge von 0.1 bis 50 Gewichts-%, bevorzugt 0.5 bis 30 Gewichts-%, und insbesondere 1 bis 20 Gewichts-%, bezogen auf die zweikomponentige Isocyanat-haltige Zusammensetzung, vorhanden.

Es ist vorteilhaft,wenn die zweikomponentige Isocyanat-haltige Zusammensetzung mindestens einen Katalysator **KAT-2** enthält.Als Katalysator **KAT-2** geeignet sind Verbindungen, welche die Aushärtung der Zusammensetzung beschleunigen. Als geeignete Katalysatoren **KAT-2** genannt werden sollen einerseits die bereits genannten Katalysatoren **KAT-1,** sowie weitere Katalysatoren, beispielsweise Verbindungen von Zink, Mangan, Eisen, Chrom, Cobalt, Kupfer, Nickel, Molybdän, Blei, Cadmium, Quecksilber, Antimon, Vanadium, Titan, Zirconium oder Kalium, wie Zink (II)-acetat, Zink(II)-2-ethylhexanoat, Zink(II)-laurat, Zink(II)-oleat, Zink(II)-naphthenat, Zink(II)-acetylacetonat, Zink(II)-Salicylat, Mangan(II)-2-ethylhexanoat, Eisen(III)-2-ethylhexanoat, Eisen(III)-acetylacetonat, Chrom(III)-2-ethylhexanoat, Cobalt(II)-naphthenat, Cobalt(II)-2-ethylhexanoat, Kupfer(II)-2-ethylhexanoat, Nickel(II)-naphthenat, Phenylquecksilber-neodecanoat, Blei(II)-acetat, Blei(II)-2-ethylhexanoat, Blei(II)-neodecanoat, Blei(II)-acetylacetonat, Aluminiumlactat, Aluminiumoleat, Aluminium(III)-acetylacetonat, Diisopropoxytitan-bis-(ethylacetoacetat), Dibutoxytitan-bis-(ethylacetoacetat), Dibutoxytitan-bis-(acetylacetonat), Kalium-acetat, Kalium-octoat; tertiäre Aminverbindungen wie Triethylamin, Tributylamin, N-Ethyl-diisopropylamin, N,N,N',N'-Tetramethyl-ethylendiamin, Pentamethyl-diethylentriamin und höhere Homologe davon, N,N,N',N'-Tetramethyl-propylendiamin, Pentamethyl-dipropylentriamin und höhere Homologe davon, N,N,N',N'-Tetramethyl-1,3-butandiamin, N,N,N',N'-Tetramethyl-1,6-hexandiamin, Bis-(dimethylamino)-methan, N,N-Dimethylbenzylamin, N,N-Dimethylcyclohexylamin, N-Methyl-dicyclohexylamin, N,N-Dimethyl-hexadecylamin, Bis-(N,N-diethylaminoethyl)-adipat, N,N-Dimethyl-2-phenylethylamin, Tris-(3-dimethylaminopropyl)-amin, 1,4-Diazabicyclo [2.2.2]octan, 1,8-Diazabicyclo [5.4.0]undec-7-en (DBU), N-Methylmorpholin, N-Ethylmorpholin, N-Cocomorpholin, N, N'-Dimethylpiperazin, N-Methyl-N'-dimethylaminoethyl-piperazin, Bis-(dimethylaminoethyl)-piperazin, 1,3,5-Tris-(dimethylaminopropyl)-hexahydrotriazin oder Bis-(2-dimethylaminoethyl)-ether; stickstoffaromatische Verbindungen wie 4-Dimethylamino-pyridin, N-Methylimidazol, N-Vinylimidazol oder 1,2-Dimethylimidazol; Amidine und Guanidine wie 1,1,3,3-Tetramethylguanidin; tertiäre Aminverbindungen enthaltend aktive Wasserstoffatome, wie Triethanolamin, Triisopropanolamin, N-Methyldiethanolamin, N,N-Dimethylethanolamin, 3-(Dimethylamino)-propyl-diisopropanolamin, Bis-(3-(dimethylamino)-propyl)-isopropanolamin, Bis-(3-dimethylaminopropyl)amin, 3-(Dimethylamino)-propyl-harnstoff, Mannich-Basen wie 2,4,6-Tris-(dimethylaminomethyl)-phenol oder 2,4,6-Tris-(3-(dimethylamino)-propylaminomethyl)-phenol, N-Hydroxypropylimidazol, N-(3-Aminopropyl)-imidazol, sowie Alkoxylierungs- und Polyalkoxylierungsprodukte dieser Verbindungen, beispielsweise Dimethylaminoethoxyethanol; organische Ammoniumverbindungen wie Benzyltrimethylammoniumhydroxid oder alkoxylierte tertiäre Amine; sogenannte "delayed action"-Katalysatoren, welche Modifizierungen bekannter Metall- oder Aminkatalysatoren darstellen, wie Umsetzungsprodukte aus tertiären Aminen und Carbonsäuren oder Phenolen, beispielsweise aus 1,4-Diazabicyclo[2.2.2]octan oder DBU und Ameisensäure oder Essigsäure; sowie Kombinationen der genannten Verbindungen, insbesondere von Metall- und Aminogruppenhaltigen Verbindungen.

Zusätzlich zur Aldimin-haltigen Verbindung der Formel (I), zum Polyisocyanat oder Isocyanat-haltigen Polyurethanpolymer **P,** zum Polyol und/oder Polyamin und zum gegebenenfalls vorhandenen Katalysator **KAT-2** kann die zweikomponentige Isocyanat-haltige Zusammensetzung weitere Bestandteile enthalten, wobei dieselben Weichmacher, Lösemittel, Füllstoffe, Katalysatoren, Reaktivverdünner und Vernetzer, latente Polyamine, Trocknungsmittel, Rheologiemodifizierer, Haftvermittler, Stabilisatoren, oberflächenaktiven Substanzen und Biocide eingesetzt werden können, welche bereits für die einkomponentige Zusammensetzung genannt wurden, sowie weitere üblicherweise in zweikomponentigen Polyurethanzusammensetzungen eingesetzte Substanzen. Die Aufteilung dieser zusätzlichen Bestandteile auf die Komponenten **K1** und **K2** erfolgt in der dem Fachmann für zweikomponentige Polyurethanzusammensetzungen bekannten Weise.

Die Komponenten **K1** und **K2** sind, getrennt voneinander aufbewahrt, jeweils lagerstabil. Insbesondere die Komponente **K1** muss unter Ausschluss von Feuchtigkeit hergestellt und gelagert werden.

Die beiden Komponenten **K1** und **K2** werden erst kurz vor der Applikation auf eine geeignete Weise miteinander vermischt, wobei darauf geachtet werden muss, dass beim Mischvorgang möglichst wenig Luft in die gemischte Zusammensetzung gelangt, und dass ein geeignetes Mischungsverhältnis eingehalten wird.Sobald die beiden Komponenten miteinander in Kontakt kommen, beginnen die in ihnen enthaltenen reaktiven Bestandteile miteinander zu reagieren und führen so zur Aushärtung der gemischten zweikomponentigen Zusammensetzung. Insbesondere reagieren die Isocyanatgruppen der Komponente **K1** mit den partiell oder vollständig hydrolysierten Aldiminogruppen der Komponente **K1** oder **K2** sowie mit den Hydroxyl- und/oder Amingruppen der Komponente **K2.** Die Aushärtung der gemischten zweikomponentigen Zusammensetzung kann bei Raumtemperatur erfolgen; gegebenenfalls kann sie auch durch Zufuhr von Wärme beschleunigt werden, insbesondere dann, wenn die Zusammensetzung langsam reagierende Polyole oder Polyisocyanate enthält, oder wenn sie thermisch latente Polyamine,wie Amin-Metallkomplexe oder mikroverkapselte Polyamine, enthält, welche erst nach Überschreiten einer Aktivierungstemperatur, beispielsweise 80-200°C, mit den Isocyanatgruppen reagieren.

Das Mischungsverhältnis zwischen den Komponenten **K1** und **K2** wird üblicherweise so gewählt, dass ein gewisser Überschuss an Isocyanatgruppen in Bezug auf mit Isocyanatgruppen reagierende Gruppen wie Aldimino-, Hydroxyl- und Aminogruppen vorhanden ist. Üblicherweise wird das Mischungsverhältnis so gewählt, dass das Verhältnis ([OH] + [NH])/[NCO] einen Wert von 0.5 bis 0.95 aufweist. Dadurch wird sichergestellt, dass die gemischte zweikomponentige Zusammensetzung zu einem polymeren Material aushärtet, wobei überschüssige Isocyanatgruppen entweder mit Feuchtigkeit aus der Komponente **K2** oder mit Feuchtigkeit aus der Luft reagieren. Ebenfalls muss darauf geachtet werden, dass zwischen dem Vermischen der Komponenten **K1** und **K2** und dem Applizieren auf eine Oberfläche eines Substrates nicht zuviel Zeit vergeht, da ein zu starkes Vorreagieren vor der Applikation das Ausbilden einer guten Haftung zum Untergrund erschwert.

Aufgrund der Geruchsfreiheit der Aldimine der Formel (XI), der Aldimin-haltigen Verbindungen der Formel (I) selbst, wie auch der bei der Hydrolyse dieser Substanzen freigesetzten Aldehyde **A** härten die beschriebenen Kunststoffvorläufer ohne Geruchsbildung aus. Sie können somit auch für Geruchsfreiheit voraussetzende Anwendungen eingesetzt werden, wie beispielsweise für Verklebungen, Abdichtungen, Beschichtungen oder Beläge im Innern von Fahrzeugen oder Gebäuden. Solche Anwendungen sind beispielsweise Klebstoffe, Dichtstoffe, Beschichtungen oder Bodenbeläge in der industriellen Fertigung oder Reparatur oder im Tief- oder Hochbau oder Innenausbau von Transportmitteln oder Bauwerken. Speziell erwähnt werden sollen Anwendungen als elastischer Klebstoff bei der Fertigung von Fahrzeugen zu Wasser oder zu Lande, insbesondere Automobile, Schiffe, Busse, Lastkraftwagen oder Züge, sowie Anwendungen als elastischer Dichtstoff bei der Fertigung von Transportmitteln oder Bauwerken.

## Beispiele

Beschreibung der Messmethoden

Die **Infrarotspektren** wurden auf einem FT-IR Gerät 1600 von Perkin-Elmer gemessen (horizontale ATR-Messeinheit mit ZnSe-Kristall); die Proben wurden unverdünnt als Filme aufgetragen. Die Absorptionsbanden sind in Wellenzahlen ($cm^{-1}$) angegeben (Messfenster: 4000-650 $cm^{-1}$).

**$^1$H-NMR-Spektren** wurden gemessen auf einem Spektrometer des Typs Bruker DPX-300 bei 300.13 MHz; die chemischen Verschiebungen $\delta$ sind angegeben in ppm relativ zu Tetramethylsilan (TMS), Kopplungskonstanten *J* sind angegeben in Hz. Die Kopplungsmuster (t, m) wurden angegeben, auch wenn es sich nur um Pseudokopplungsmuster handelt.

Die **Viskosität** wurde auf einem thermostatisierten Kegel-Platten-Viskosimeter Physica UM (Kegeldurchmesser 20 mm, Kegelwinkel 1°, Kegelspitze-Platten-Abstand 0.05 mm, Schergeschwindigkeit 10 bis 1000 $s^{-1}$) gemessen.

Der totale **Gehalt an Aldiminogruppen und freien Aminogruppen** in den hergestellten Verbindungen ("Amin-Gehalt") wurde titrimetrisch bestimmt (mit 0.1N $HClO_4$ in Eisessig, gegen Kristallviolett) und ist stets angegeben in mmol $NH_2$/g (auch wenn es sich nicht nur um primäre Aminogruppen handelt).

Aldimine der Formel (XI) enthaltend einen freien Wasserstoff

### Aldimin *AL1*

In einem Rundkolben wurden unter Stickstoffatmosphäre 40.64 g (0.143 mol) 2,2-Dimethyl-3-lauroyloxy-propanal vorgelegt. Unter kräftigem Rühren wurden aus einem Eintropftrichter innerhalb von 5 Minuten 11.68 g (0.133 mol) N-Methyl-1,3-propandiamin zugegeben, wobei die Temperatur des Reaktionsgemisches auf 38°C anstieg. Danach wurden die flüchtigen Bestandteile im Vakuum entfernt (10 mbar, 80°C). Man erhielt 49.8 g einer farblosen, bei Raumtemperatur dünnflüssigen, klaren und geruchlosen Flüssigkeit, die einen Amin-Gehalt von 5.20 mmol $NH_2$/g aufwies. Das Produkt liegt grösstenteils in der offenkettigen (Aldimin-) Form vor. IR:3329(N-H),2954sh,2922,2852,789,1736 (C=O),1668(C=N),1466,1419sh,1392,1374, 1348, 1300, 1249, 1234, 1160, 1112, 1069, 1058, 1021, 996, 938, 886, 876, 820, 722. $^1$H-NMR ($CDCl_3$, 300 K): $\delta$ 7.53 (*s,* 1 H, CH=N), 4.01 (*s,* 2 H, $CH_2O$), 3.44 (*t,* 2 H, CH=NC$H_2$$CH_2$), 2.58 (*t,* 2 H, NHC$H_2$), 2.42 (*s,* 3 H, C$H_3$NH), 2.30 (*t,* 2 H, $CH_2CO$), 1.76 (*t,* 2 H, CH=N$CH_2$C$H_2$), 1.61 (*m,* 3 H, C$H_2$$CH_2$CO und $CH_3$N*H*$CH_2$), 1.27 (*m,* 16 H, $CH_3$-(C$H_2$)$_8$-$CH_2CH_2$CO), 1.10 (*s,* 6 H, C(C$H_3$)$_2$-$CH_2$O), 0.89 (*t,* 3H, C$H_3$-($CH_2$)$_{10}$-CO).

### Aldimin *AL2*

In einem Rundkolben wurden unter Stickstoffatmosphäre 30.13 g (0.106 mol) 2,2-Dimethyl-3-lauroyloxy-propanal vorgelegt. Unter kräftigem Rühren wurden aus einem Eintropftrichter innerhalb von 5 Minuten 15.00 g (0.096 mol) N-Cyclohexyl-1,3-propandiamin zugegeben, wobei die Temperatur des Reaktionsgemisches auf 36°C anstieg. Danach wurden die flüchtigen Bestandteile im Vakuum entfernt (10 mbar, 80°C). Man erhielt 43.2 g einer farblosen, bei Raumtemperatur dünnflüssigen, klaren und geruchlosen Flüssigkeit, die einen Amin-Gehalt von 4.39 mmol $NH_2$/g aufwies. Das Produkt liegt grösstenteils in der offenkettigen (Aldimin-) Form vor.

IR: 3308 (N-H), 2921, 2851, 2659, 1737 (C=O), 1668 (C=N), 1465, 1449, 1418sh, 1393, 1366, 1346, 1301, 1248, 1158, 1111, 1068, 1020, 1002, 938, 888, 845, 797, 721. $^1$H-NMR ($CDCl_3$, 300 K): $\delta$ 7.53 (*s,* 1 H, CH=N), 4.01 (*s,* 2 H, $CH_2O$), 3.43 (*t,* 2 H, CH=N$CH_2CH_2$), 2.65 (*t,* 2.H, NHC$H_2$), 2.40 (*s,* 1 H, Cy-C$^1$*H*NH), 2.29 (*t,* 2 H, $CH_2CO$), 1.86

(*m,* 2 H, 2 Cy-H), 1.72 (*m,* 4 H, 2 Cy-H und $CH{=}NCH_2CH_2$), 1.60 (*m,* 3 H, $CH_2CH_2CO$ und $CH_3NHCH_2$), 1.26 (*m,* 22 H, $CH_3$-$(CH_2)_8$-$CH_2CH_2CO$ und 6 Cy-H), 1.09 (*s,* 6 H, $C(CH_3)_2$-$CH_2O$), 0.88 (*t,* 3 H, $CH_3$-$(CH_2)_{10}$-CO).

**Aldimin *AL3***

In einem Rundkolben wurden unter Stickstoffatmosphäre 69.31 g (0.244 mol) 2,2-Dimethyl-3-lauroyloxy-propanal vorgelegt. Unter kräftigem Rühren wurden aus einem Eintropftrichter innerhalb von 5 Minuten 14.72 g (0.112 mol) Dipropylentriamin zugegeben, wobei die Temperatur des Reaktionsgemisches auf 36˚C anstieg. Danach wurden die flüchtigen Bestandteile im Vakuum entfernt (10 mbar, 80˚C). Man erhielt 79.7 g einer farblosen, bei Raumtemperatur dünnflüssigen, klaren und geruchlosen Flüssigkeit, die einen Amin-Gehalt von 4.17 mmol $NH_2$/g aufwies. Das Produkt liegt grösstenteils in der offenkettigen (Aldimin-) Form vor.
IR:3308(N-H), 2952sh, 2921,2851,1737(C=O), 1667(C=N), 1466, 1418sh, 1393, 1373, 1348, 1301, 1248, 1234, 1159, 1111, 1070, 1019, 1001, 936, 875, 722.
$^1$H-NMR ($CDCl_3$, 300 K): δ 7.53 (*s,* 2 H, CH=N), 4.01 (*s,* 4 H, $CH_2O$), 3.42 (*t,* 4 H, $CH{=}NCH_2CH_2$), 2.61 (*t,* 4 H, $NHCH_2$), 2.29 (*t,* 4 H, $CH_2CO$), 1.73 (*m,* 4 H, $CH{=}NCH_2CH_2$), 1.59 (*m,* 5 H, $CH_2CH_2CO$ und $CH_2NHCH_2$), 1.25 (*m,* 32 H, $CH_3$-$(CH_2)_8$-$CH_2CH_2CO$), 1.09 (*s*, 12 H, $C(CH_3)_2$-$CH_2O$), 0.87 (*t,* 6 H, $CH_3$-$(CH_2)_{10}$-CO).

**Aldimin *AL4***

In einem Rundkolben wurden unter Stickstoffatmosphäre 34.15 g (0.120 mol) 2,2-Dimethyl-3-lauroyloxy-propanal vorgelegt. Unter kräftigem Rühren wurden aus einem Eintropftrichter innerhalb von 5 Minuten 12.02 g (0.056 mol) Bis-hexamethylentriamin (BHMT-HP; Invista) zugegeben, wobei die Temperatur des Reaktionsgemisches auf 35˚C anstieg. Danach wurden die flüchtigen Bestandteile im Vakuum entfernt (10 mbar, 80˚C). Man erhielt 43.6 g einer farblosen, bei Raumtemperatur dünnflüssigen, klaren und geruchlosen Flüssigkeit, die einen Amin-Gehalt von 3.68 mmol $NH_2$/g aufwies. Das Produkt liegt grösstenteils in der offenkettigen (Aldimin-) Form vor.
IR: 2922, 2851, 1737 (C=O), 1668 (C=N), 1465, 1417, 1393, 1373, 1340, 1248, 1234, 1159, 1111, 1020, 1003, 933, 870, 722.
$^1$H-NMR ($CDCl_3$, 300 K): δ 7.52 (*s,* 2 H, CH=N), 4.02 (*s,* 4 H, $CH_2O$), 3.36 (*t,* 4 H, $CH{=}NCH_2CH_2$), 2.59 (*t,* 4 H, $NHCH_2$), 2.29 (*t,* 4 H, $CH_2CO$), 1.76-1.51 (*m,* 13 H, $CH{=}NCH_2CH_2$, $NHCH_2CH_2$, $CH_2CH_2CO$ und $CH_2NHCH_2$), 1.27 (*m,* 40 H, $CH_3$-$(CH_2)_8$-$CH_2CH_2CO$ und $NHCH_2CH_2CH_2$), 1.10 (*s,* 12 H, $C(CH_3)_2$-$CH_2O$), 0.88 (*t,* 6 H, $CH_3$-$(CH_2)_{10}$-CO).

**Aldimin *AL5***

In einem Rundkolben wurden unter Stickstoffatmosphäre 30.28 g (0.106 mol) 2,2-Dimethyl-3-lauroyloxy-propanal vorgelegt. Unter kräftigem Rühren wurden aus einem Eintropftrichter innerhalb von 5 Minuten 5.00 g (0.049 mol) Diethylentriamin zugegeben. Danach wurden die flüchtigen Bestandteile im Vakuum entfernt (10 mbar, 80˚C). Man erhielt 33.1 g einer farblosen, bei Raumtemperatur dünnflüssigen, klaren und geruchlosen Flüssigkeit, die einen Amin-Gehalt von 4.07 mmol $NH_2$/g aufwies. Das Produkt liegt grösstenteils in der offenkettigen (Aldimin-) Form vor.
IR: 3348 (N-H), 2952, 2921, 2852, 1735 (C=O), 1668 (C=N), 1632, 1465, 1417, 1393, 1373, 1345, 1248, 1232, 1158, 1110, 1056, 1022, 1005, 986, 931, 903, 875, 820, 721.

**Aldimin *AL6***

In einem Rundkolben wurden unter Stickstoffatmosphäre 20.97 g (0.074 mol) 2,2-Dimethyl-3-lauroyloxy-propanal vorgelegt. Unter kräftigem Rühren wurden aus einem Eintropftrichter innerhalb von 5 Minuten 10.00 g (0.067 mol) Triethylenglykol-monoamin (Jeffamine® XTA-250; Huntsman) zugegeben, wobei die Temperatur des Reaktionsgemisches auf 33˚C anstieg. Danach wurden die flüchtigen Bestandteile im Vakuum entfernt (10 mbar, 80˚C). Man erhielt 29.5 g einer farblosen, bei Raumtemperatur dünnflüssigen, klaren und geruchlosen Flüssigkeit, die einen Amin-Gehalt von 2.21 mmol $NH_2$/g aufwies. Das Produkt liegt grösstenteils in der offenkettigen (Aldimin-) Form vor.
IR: 3444br (O-H), 2952sh, 2921, 2852, 1736 (C=O), 1668 (C=N), 1466, 1418, 1394, 1374, 1366, 1350, 1301sh, 1248, 1145sh, 1116, 1067, 1023sh, 998sh, 932, 890, 829,722. $^1$H-NMR ($CDCl_3$, 300 K): δ 7.59 (*s,* 1 H, CH=N), 4.03 (*s,* 2 H, $CH_2O$), 3.79-3.59 (*m,* 12 H, $HOCH_2CH_2OCH_2CH_2OCH_2CH_2N$), 3.47 (*s,* 1 H, $HOCH_2$), 2.31 (*t,* 2 H, $CH_2CO$), 1.61 (*m,* 2 H, $CH_2CH_2CO$), 1.27 (*m,* 16 H, $CH_3$-$(CH_2)_8$-$CH_2CH_2CO$), 1.11 (*s,* 6 H, $C(CH_3)_2$-$CH_2O$), 0.87 (*t,* 3 H, $CH_3$-$(CH_2)_{10}$-CO).

**Aldimin *AL7***

In einem Rundkolben wurden unter Stickstoffatmosphäre 34.48 g (0.121 mol) 2,2-Dimethyl-3-lauroyloxy-propanal vorgelegt. Unter kräftigem Rühren wurden aus einem Eintropftrichter innerhalb von 15 Minuten 20.00 g (0.117

mol) Isophorondiamin (Vestamin® IPD, Degussa) zugegeben. Danach wurden die flüchtigen Bestandteile im Vakuum entfernt (10 mbar, 80˚C). Zum so erhaltenen klaren, farblosen Öl gab man bei Raumtemperatur 25.25 g (0.121 mol) Isobornylacrylat (SR-506, Sartomer). Man liess 30 Minuten bei Raumtemperatur rühren, erwärmte die Mischung dann auf 85˚C und hielt sie während 24 Stunden bei dieser Temperatur. Darauf wurden die flüchtigen Bestandteile im Hochvakuum entfernt (100˚C). Man erhielt 72.0 g einer farblosen, bei Raumtemperatur dünnflüssigen, klaren und geruchlosen Flüssigkeit, die einen Amin-Gehalt von 3.09 mmol $NH_2$/g aufwies. Das Produkt liegt grösstenteils in der offenkettigen (Aldimin-) Form vor.
IR: 3322 (N-H), 2950, 2923, 2871, 2852, 1732 (C=O), 1668 (C=N), 1457, 1418sh, 1388sh, 1377, 1364, 1310, 1294, 1248, 1196, 1165, 1110, 1053, 1015, 987, 969, 942, 931sh, 914, 893, 863, 840, 796, 722.

**Aldimin *AL8* (Vergleich)**

In einem Rundkolben wurden unter Stickstoffatmosphäre 48.18 g (0.243 mol) 3-Phenoxybenzaldehyd vorgelegt. Unter kräftigem Rühren wurden aus einem Eintropftrichter innerhalb von 5 Minuten 20.00 g (0.227 mol) N-Methyl-1,3-propandiamin zugegeben, wobei die Temperatur des Reaktionsgemisches auf 40˚C anstieg. Danach wurden die flüchtigen Bestandteile im Vakuum entfernt (10 mbar, 80˚C). Man erhielt 63.7 g einer hellgelben, bei Raumtemperatur dünnflüssigen, klaren und stark riechenden Flüssigkeit, die einen Amin-Gehalt von 7.08 mmol $NH_2$/g aufwies. Das Produkt liegt mehrheitlich in der cyclischen (Tetrahydropyrimidin-) Form vor.
IR: 3270 (N-H), 3060, 3036, 2978, 2940, 2837, 2773, 2692, 1935, 1865, 1778, 1702, 1645, 1582, 1483, 1456, 1442, 1418, 1370, 1353, 1308, 1236, 1210, 1188, 1163, 1128, 1108, 1072, 1053, 1023, 990, 964, 937, 917, 900, 889, 877, 839, 775, 748, 690. $^1$H-NMR ($CDCl_3$, 300 K): δ 7.42-7.28 (m, 5 Ar-H), 7.16-7.01 (m, 4 Ar-H), 3.74 (*s,* 1 H, Ar-C*H*(NH)N), 3.14 (*m,* 2 H, $HNCH^{eq}H^{ax}$ und $CH_3NCH^{eq}H^{ax}$), 2.78 (*m,* 1 H, $HNCH^{eq}H^{ax}$), 2.35 (*m,* 1 H, $CH_3NCH^{eq}H^{ax}$), 2.06 (*s,* 3 H, $CH_3N$), 1.90 (*m,* 1 H, $CH_3NCH_2CH^{eq}H^{ax}$), 1.58 (*m,* 2 H, $CH_3NCH_2CH^{eq}H^{ax}$ und $HNCH_2$).

**Aldimin *AL9***

In einem Rundkolben wurden unter Stickstoffatmosphäre 28.06 g (0.099 mol) 2,2-Dimethyl-3-lauroyloxy-propanal vorgelegt. Unter kräftigem Rühren wurden aus einem Eintropftrichter innerhalb von 3 Minuten 10.00 g (0.095 mol) 2-(2-Aminoethoxy)-ethanol (Diglycolamine® Agent; Huntsman) zugegeben, wobei die Temperatur des Reaktionsgemisches auf 40˚C anstieg. Danach wurden die flüchtigen Bestandteile im Vakuum entfernt (10 mbar, 80˚C). Man erhielt 36.3 g einer farblosen, bei Raumtemperatur dünnflüssigen, klaren und geruchlosen Flüssigkeit, die einen Amin-Gehalt von 2.58 mmol $NH_2$/g aufwies. Das Produkt liegt grösstenteils in der offenkettigen (Aldimin-) Form vor.
IR: 3435br (O-H), 2954sh, 2922, 2852, 1736 (C=O), 1668 (C=N), 1466, 1418, 1394, 1375, 1248, 1233, 1160, 1127, 1062, 1022, 933, 893, 813, 721.
$^1$H-NMR ($CDCl_3$, 300 K): δ 7.59 (*s,* 1 H, CH=N), 4.03 (*s,* 2 H, $CH_2O$), 3.71 (*m,* 4 H, $HOCH_2CH_2OCH_2CH_2N$) 3.58 (m, 4 H, $HOCH_2CH_2OCH_2CH_2N$), 2.44 (br *s,* 1 H, $HOCH_2$), 2.30 (*t*, 2 H, $CH_2CO$), 1.61 (*m,* 2 H, $CH_2CH_2CO$), 1.26 (*m,* 16 H, $CH_3$-$(CH_2)_8$-$CH_2CH_2CO$), 1.11 (*s,* 6 H, $C(CH_3)_2$-$CH_2O$), 0.88 (*t,* 3 H, $CH_3$-$(CH_2)_{10}$-CO).

**Aldimin *AL10***

In einem Rundkolben wurden unter Stickstoffatmosphäre 34.51 g (0.121 mol) 2,2-Dimethyl-3-lauroyloxy-propanal vorgelegt. Unter kräftigem Rühren wurden aus einem Eintropftrichter innerhalb von 5 Minuten 33.39 g N-Oleyl-1,3-propandiamin (Duomeen® O, Akzo Nobel; Aminzahl = 337 mg KOH/g) zugegeben, wobei die Temperatur des Reaktionsgemisches auf 48˚C anstieg. Danach wurden die flüchtigen Bestandteile im Vakuum entfernt (10 mbar, 80˚C). Man erhielt 65.7 g einer farblosen, bei Raumtemperatur dünnflüssigen, klaren und geruchlosen Flüssigkeit, die einen Amin-Gehalt von 3.07 mmol $NH_2$/g aufwies. Das Produkt liegt grösstenteils in der offenkettigen (Aldimin-) Form vor. IR: 3307 (N-H), 3001sh, 2954sh, 2921, 2851, 1739 (C=O), 1668 (C=N), 1464, 1393,1375,1347,1301,1248,1158, 1114, 1067, 1020, 1000, 968, 935, 889, 721. $^1$H-NMR ($CDCl_3$, 300 K): δ 7.53 (*t, J* = 1.2) und 7.51 (*s*) (total 1 H (Verhältnis ca. 0.85 / 0.15), CH=N), 5.34 (*m*, 2 H, $CH_2CH$=$CHCH_2$), 4.01 (*s*, 2 H, $CH_2O$), 3.43 (*t*, 2 H, CH=$NCH_2CH_2$), 2.60 (*m*, 4 H, CH=$NCH_2CH_2CH_2$ und $NHCH_2$), 2.30 (*t*, 2 H, $CH_2CO$), 2.01 (m, 4H, $CH_2CH$=$CHCH_2$), 1.75 (*m,* 2 H, CH=$NCH_2CH_2$), 1.60 (*m,* 3 H, $CH_2CH_2CO$ und $CH_2NHCH_2$), 1.47 (*m,* 2 H, $CH_2NHCH_2CH_2$), 1.26 (*m,* 38 H, übrige $CH_2$-Gruppen), 1.09 (*s,* 6 H, $C(CH_3)_2$-$CH_2O$), 0.88 (*t,* 6 H, beide $CH_3CH_2CH_2$).

**Aldimin *AL11***

In einem Rundkolben wurden unter Stickstoffatmosphäre 40.00 g (0.141 mol) 2,2-Dimethyl-3-lauroyloxy-propanal vorgelegt. Unter kräftigem Rühren wurden aus einem Eintropftrichter innert 5 Minuten 24.00 g (0.128 mol) N-(2-Ethylhexyl)-1,3-propandiamin (BASF) zugegeben, die Mischung auf 80˚C erwärmt und gleichzeitig die flüchtigen Bestandteile im Vakuum entfernt (10 mbar). Man erhielt 61.5 g einer farblosen, bei Raumtemperatur dünnflüssigen, klaren und geruchlosen Flüssigkeit, die einen Amin-Gehalt von 4.12 mmol $NH_2$/g aufwies. Das Produkt liegt grösstenteils in der offenkettigen (Aldimin-) Form vor.
IR: 3322(N-H),2955,2922,2870sh,2852,2824sh, 1738 (C=O), 1668 (C=N), 1464,1393, 1376, 1342, 1300, 1248, 1235, 1157, 1114, 1069, 1020, 1000, 935, 894, 873, 766, 723.

**Aldimin *AL12***

In einem Rundkolben wurden unter Stickstoffatmosphäre 35.00 g (0.123 mol) 2,2-Dimethyl-3-lauroyloxy-propanal vorgelegt. Unter kräftigem Rühren wurden innerhalb von 5 Minuten 36.31 g 50˚C warmes N-Talgalkyl-1,3-propandiamin (Duomeen® T, Akzo Nobel; Aminzahl = 346 mg KOH/g) zugegeben, die Mischung auf 80˚C erwärmt und gleichzeitig die flüchtigen Bestandteile im Vakuum entfernt (10 mbar). Man erhielt 69.2 g eines schmutzigweissen, bei Raumtemperatur festen und geruchlosen Körpers, der einen Amin-Gehalt von 3.20 mmol $NH_2$/g aufwies. Das Produkt liegt grösstenteils in der offenkettigen (Aldimin-) Form vor.
IR: 3316 (N-H), 2954sh, 2919, 2851, 2815sh, 1739 (C=O), 1668 (C=N), 1464, 1393, 1375, 1347, 1300, 1248, 1233, 1158, 1128sh, 1114, 1068, 1021, 1000, 968, 936, 917sh, 889, 873, 721.

<u>Aldimin-haltige Verbindungen Formel (I)</u>

**Beispiel 1 (Aldimin-haltige Verbindung *AV1*)**

In einem Rundkolben wurden unter Stickstoffatmosphäre 1.74 g (13.9 mmol NCO) 4,4'-Diphenylmethan-diisocyanat (MDI; Desmodur® 44 MC L, Bayer) vorgelegt und auf 50 ˚C erwärmt. Aus einem Eintropftrichter wurden unter gutem Rühren innerhalb von 5 Minuten 10.00 g (13.9 mmol) Aldimin ***AL3*** zugegeben und die Mischung während einer Stunde bei 50 ˚C gerührt. Man erhielt eine farblose, bei Raumtemperatur dickflüssige, klare und geruchlose Flüssigkeit mit einem Amin-Gehalt von 2.37 mmol $NH_2$/g, die auf einem angefeuchteten pH-Papier neutral reagierte.
IR: 3300 (N-H), 2952sh, 2922, 2851, 1735 (C=O), 1664 (C=N), 1647sh, 1595, 1527sh, 1513, 1466, 1416, 1395, 1375, 1305, 1244, 1215, 1196, 1162, 1112, 1056, 1018, 1000, 939, 918sh, 851, 813, 777, 751, 721.

**Beispiel 2 (Aldimin-haltige Verbindung *AV2*)**

In einem Rundkolben wurden unter Stickstoffatmosphäre 3.47 g (27.7 mmol NCO) 4,4'-Diphenylmethan-diisocyanat (MDI; Desmodur® 44 MC L, Bayer) vorgelegt und auf 50˚C erwärmt. Aus einem Eintropftrichter wurden unter gutem Rühren innerhalb von 5 Minuten 10.00 g (13.9 mmol) Aldimin ***AL3*** zugegeben und die Mischung während einer Stunde bei 50˚C gerührt. Man erhielt eine blassgelbe, bei Raumtemperatur dickflüssige, klare und geruchlose Flüssigkeit, die auf einem angefeuchteten pH-Papier neutral reagierte.
IR: 3308 (N-H), 2954sh, 2922, 2852, 2266 (N=C=O), 1735 (C=O), 1665 (C=N), 1596, 1526sh, 1514, 1467, 1415, 1395, 1374, 1306, 1244, 1216, 1197, 1162, 1110, 1059, 1018, 1000, 940, 918sh, 854, 813, 781, 751, 721.

**Beispiel 3 (Aldimin-haltige Verbindung *AV3*)**

In einem Rundkolben wurden unter Stickstoffatmosphäre 12.94 g (103.4 mmol NCO) 4,4'-Diphenylmethan-diisocyanat (MDI; Desmodur® 44 MC L, Bayer) vorgelegt und auf 50 ˚C erwärmt. Aus einem Eintropftrichter wurden unter gutem Rühren innerhalb von 10 Minuten 42.16 g (51.7 mmol) Aldimin ***AL4*** zugegeben und die Mischung während einer Stunde bei 50 ˚C gerührt. Man erhielt eine hellgelbe, bei Raumtemperatur dickflüssige, klare und geruchlose Flüssigkeit, die auf einem angefeuchteten pH-Papier neutral reagierte.
IR: 3336 (N-H), 2922, 2852, 2265 (N=C=O), 1736 (C=O), 1666 (C=N), 1640, 1594, 1513, 1488, 1465, 1416, 1394, 1373, 1307, 1237, 1169, 1110, 1065, 1018, 1000sh, 932, 918sh, 848, 812, 776, 754, 723.

**Beispiel 4 (Aldimin-haltige Verbindung *AV4*)**

In einem Rundkolben wurden unter Stickstoffatmosphäre 10.00 g (51.4 mmol NCO) 1,6-Hexamethylendiisocyanat-Trimerisat (Desmodur® N-3300, Bayer; NCO-Gehalt = 21.61 Gewichts-%) in 29.79 g trockenem Diisodecylphthalat (DIDP; Palatinol® Z, BASF) gelöst. Aus einem Eintropftrichter wurden unter gutem Rühren bei Raumtemperatur innerhalb von 10 Minuten 19.79 g (102.9 mmol) Aldimin ***AL1*** zugegeben und die Mischung während einer Stunde gerührt. Man erhielt eine farblose, bei Raumtemperatur dünnflüssige, klare und geruchlose Flüssigkeit mit einem Amin-Gehalt von 0.87 mmol $NH_2$/g, die auf einem angefeuchteten pH-Papier neutral reagierte.
IR: 3423 (N-H), 3326 (N-H), 2954, 2924, 2853, 1726 (C=O), 1688, 1650, 1600, 1579, 1529, 1462, 1377, 1335, 1272,

1164, 1121, 1072, 1039, 985, 965, 948, 764, 742, 704.

**Beispiel 5 (Aldimin-haltige Verbindung *AV5*)**

In einem Rundkolben wurden unter Stickstoffatmosphäre 10.00 g (51.4 mmol NCO) 1,6-Hexamethylendiisocyanat-Trimerisat (Desmodur® N-3300, Bayer; NCO-Gehalt = 21.61 Gewichts-%) in 47.05 g trockenem Ethylacetat gelöst. Aus einem Eintropftrichter wurden unter gutem Rühren bei Raumtemperatur innerhalb von 10 Minuten 37.05 g (102.9 mmol) Aldimin ***AL3*** zugegeben und die Mischung während einer Stunde gerührt. Man erhielt eine farblose, bei Raumtemperatur dünnflüssige, klare und geruchlose Flüssigkeit mit einem Amin-Gehalt von 1.11 mmol $NH_2$/g, die auf einem angefeuchteten pH-Papier neutral reagierte.
IR: 3422 (N-H), 3308 (N-H), 2954, 2924, 2853, 1727 (C=O), 1689, 1651, 1600, 1579, 1528, 1462, 1377, 1334, 1272, 1161, 1121, 1072, 1039, 995, 948, 870, 764, 742, 704.

**Beispiel 6 (Aldimin-haltige Verbindung *AV6*)**

79.21 g (40.2 mmol OH) Polyoxypropylen-Diol (Acclaim® 4200 N, Bayer; OH-Zahl 28.5 mg KOH/g), 10.79 g (43.1 mmol) 4,4'-Methylendiphenyldiisocyanat (MDI; Desmodur® 44 MC L, Bayer) und 10.00 g Diisodecylphthalat (DIDP; Palatinol® Z, BASF) wurden bei 80 °C zu einem NCO-terminierten Polyurethanpolymeren mit einem Gehalt an freien Isocyanat-Gruppen von 1.86 Gewichts-% und einer Viskosität bei 20 °C von 24 Pa·s umgesetzt. Zu diesem Polymer wurden bei Raumtemperatur 8.51 g (22.1 mmol) Aldimin ***AL1*** gegeben und die Mischung mittels eines Zentrifugalmischers (SpeedMixer™ DAC 150, FlackTek Inc.) innig vermengt. Man erhielt eine klare, homogene und geruchlose Flüssigkeit mit einer Viskosität bei 20 °C von 40 Pa·s.

**Beispiel 7 (Aldimin-haltige Verbindung *AV7*)**

79.21 g (40.2 mmol OH) Polyoxypropylen-Diol (Acclaim® 4200 N, Bayer; OH-Zahl 28.5 mg KOH/g), 10.79 g (43.1 mmol) 4,4'-Methylendiphenyldiisocyanat (MDI; Desmodur® 44 MC L, Bayer) und 10.00 g Diisodecylphthalat (DIDP; Palatinol® Z, BASF) wurden bei 80 °C zu einem NCO-terminierten Polyurethanpolymeren mit einem Gehalt an freien Isocyanat-Gruppen von 1.86 Gewichts-% und einer Viskosität bei 20 °C von 24 Pa·s umgesetzt. Zu diesem Polymer wurden bei Raumtemperatur 10.62 g (14.8 mmol) Aldimin ***AL3*** gegeben und die Mischung mittels eines Zentrifugalmischers (SpeedMixer™ DAC 150, FlackTek Inc.) innig vermengt. Man erhielt eine klare, homogene und geruchlose Flüssigkeit mit einer Viskosität bei 20 °C von 29 Pa·s.

**Beispiel 8 (Aldimin-haltige Verbindung *AV8*)**

79.21 g (40.2 mmol OH) Polyoxypropylen-Diol (Acclaim® 4200 N, Bayer; OH-Zahl 28.5 mg KOH/g), 10.79 g (43.1 mmol) 4,4'-Methylendiphenyldiisocyanat (MDI; Desmodur® 44 MC L, Bayer) und 10.00 g Diisodecylphthalat (DIDP; Palatinol® Z, BASF) wurden bei 80 °C zu einem NCO-terminierten Polyurethanpolymeren mit einem Gehalt an freien Isocyanat-Gruppen von 1.86 Gewichts-% und einer Viskosität bei 20 °C von 24 Pa·s umgesetzt. Zu diesem Polymer wurden bei Raumtemperatur 17.03 g (44.3 mmol) Aldimin ***AL1*** gegeben und die Mischung mittels eines Zentrifugalmischers (SpeedMixer™ DAC 150, FlackTek Inc.) innig vermengt. Nach 10 Minuten war die NCO-Bande im FT-IR-Spektrum (bei 2265 $cm^{-1}$) nicht mehr nachweisbar. Man erhielt eine klare, homogene und geruchlose Flüssigkeit mit einer Viskosität bei 20 °C von 52 Pa·s und einem Amin-Gehalt von 0.38 mmol $NH_2$/g.

**Beispiel 9 (Aldimin-haltige Verbindung *AV9*)**

79.21 g (40.2 mmol OH) Polyoxypropylen-Diol (Acclaim® 4200 N, Bayer; OH-Zahl 28.5 mg KOH/g), 10.79 g (43.1 mmol) 4,4'-Methylendiphenyldiisocyanat (MDI; Desmodur® 44 MC L, Bayer) und 10.00 g Diisodecylphthalat (DIDP; Palatinol® Z, BASF) wurden bei 80 °C zu einem NCO-terminierten Polyurethanpolymeren mit einem Gehalt an freien Isocyanat-Gruppen von 1.86 Gewichts-% und einer Viskosität bei 20 °C von 24 Pa·s umgesetzt. Zu diesem Polymer wurden bei Raumtemperatur 31.85 g (44.3 mmol) Aldimin ***AL3*** gegeben und die Mischung mittels eines Zentrifugalmischers (SpeedMixer™ DAC 150, FlackTek Inc.) innig vermengt. Nach 10 Minuten war die NCO-Bande im FT-IR-Spektrum (bei 2265 $cm^{-1}$) nicht mehr nachweisbar. Man erhielt eine klare, homogene und geruchlose Flüssigkeit mit einer Viskosität bei 20 °C von 44 Pa·s und einem Amin-Gehalt von 0.67 mmol $NH_2$/g.

**Beispiel 10 (Aldimin-haltige Verbindung *AV10*)**

25.97 g (13.2 mmol OH) Polyoxypropylen-Diol (Acclaim® 4200 N, Bayer; OH-Zahl 28.5 mg KOH/g), 51.95 g

(32.4 mmol OH) Polyol Caradol® MD34-02 (Polypropylenoxidpolyethylenoxid-Triol, OH-Zahl 35.0 mg KOH/g; Shell), 12.08 g (48.3 mmol) 4,4'-Methylendiphenyldiisocyanat (MDI; Desmodur® 44 MC L, Bayer) und 10.00 g Diisodecylphthalat (DIDP; Palatinol® Z, BASF) wurden bei 80 °C zu einem NCO-terminierten Polyurethanpolymeren mit einem Gehalt an freien Isocyanat-Gruppen von 2.07 Gewichts-% und einer Viskosität bei 20 °C von 48 Pa·s umgesetzt. Zu diesem Polymer wurden bei Raumtemperatur 18.95 g (49.3 mmol) Aldimin ***AL1*** gegeben und die Mischung mittels eines Zentrifugalmischers (SpeedMixer™ DAC 150, FlackTek Inc.) innig vermengt. Nach 10 Minuten war die NCO-Bande im FT-IR-Spektrum (bei 2265 $cm^{-1}$) nicht mehr nachweisbar. Man erhielt eine klare, homogene und geruchlose Flüssigkeit mit einer Viskosität bei 20 °C von 89 Pa·s und einem Amin-Gehalt von 0.41 mmol $NH_2$/g.

**Beispiel 11 (Aldimin-haltige Verbindung *AV11*)**

In einem Rundkolben wurden unter Stickstoffatmosphäre 5.85 g (0.052 mol NCO) Isophorondiisocyanat (Vestanat® IPDI, Degussa) vorgelegt. Unter Rühren wurden bei Raumtemperatur aus einem Eintropftrichter innerhalb von 5 Minuten 10.00 g (0.026 mol) Aldimin ***AL1*** zugegeben und die Mischung während 30 Minuten gerührt. Zum so erhaltenen klaren, farblosen Öl gab man bei Raumtemperatur 3.38 g (0.026 mol) 2-Hydroxyethylmethacrylat (HEMA; Bisomer® HEMA, Laporte). Man liess 10 Minuten rühren, gab dann 2 mg Dibutylzinndilaurat zu, erwärmte die Mischung auf 75 °C und hielt sie solange bei dieser Temperatur, bis die Isocyanatbande im FT-IR-Spektrum (bei 2253 $cm^{-1}$) verschwunden war (1 Stunde). Man erhielt eine farblose, dickflüssige, klare und geruchlose Flüssigkeit, die einen Amin-Gehalt von 1.32 mmol $NH_2$/g aufwies. IR:3334(N-H),2952,2923,2852,1719(C=O),1663sh(C=N),1636(C=C,C=O),1527, 1459, 1377, 1364, 1341, 1296, 1234, 1164, 1060, 1044, 1017, 939, 891, 869, 814, 770, 721.

**Beispiel 12 (Aldimin-haltige Verbindung *AV12*)**

In einem Rundkolben wurden unter Stickstoffatmosphäre 5.48 g (26.0 mmol NCO) m-Isopropenyl-$\alpha$,$\alpha$-dimethylbenzylisocyanat (m-TMI; Cytec) vorgelegt. Unter Rühren wurden bei Raumtemperatur aus einem Eintropftrichter innert 5 Minuten 10.00 g (26.0 mmol) Aldimin ***AL1*** zugegeben und die Mischung solange gerührt,bis die Isocyanatbande im FT-IR-Spektrum (bei 2255 $cm^{-1}$) verschwunden war (30 Minuten). Man erhielt eine farblose, dickflüssige, klare und geruchlose Flüssigkeit, die einen Amin-Gehalt von 1.66 mmol $NH_2$/g aufwies. IR: 3361 (N-H), 2953sh, 2922, 2852, 1736 (C=O), 1689, 1658, 1646, 1600, 1578, 1523, 1483, 1465, 1457, 1440sh, 1417sh, 1375, 1361sh, 1346sh, 1302, 1241, 1218sh, 1162, 1111, 1051, 1015, 1003, 938, 886, 797, 764, 722, 695.

**Beispiel 13 (Aldimin-haltige Verbindung *AV13*)**

In einem Rundkolben wurden unter Stickstoffatmosphäre eine Mischung von 2.57 g (8.7 mmol) Trimethylolpropan-triacrylat (TMPTA; SR-351, Sartomer) und 10.00 g (26.0 mmol) Aldimin ***AL1*** auf 90 °C erwärmt und solange auf dieser Temperatur gehalten, bis die Acrylbande im FT-IR-Spektrum ($\delta_{C=C\text{-}H\ oop}$ bei 808 $cm^{-1}$) verschwunden war (3 Stunden). Man erhielt eine farblose, niedrigviskose, klare und geruchlose Flüssigkeit, die einen Amin-Gehalt von 4.06 mmol $NH_2$/g aufwies.
IR: 2952sh, 2922, 2851, 2795sh, 2771sh, 1736 (C=O), 1667 (C=N), 1464, 1419, 1392sh, 1375, 1345, 1300, 1248, 1163, 1120, 1054, 1032, 1009, 934, 876, 783, 722.

**Beispiel 14 (Aldimin-haltige Verbindung *AV14*)**

In einem Rundkolben wurden unter Stickstoffatmosphäre eine Mischung von 1.37 g (4.6 mmol) Trimethylolpropan-triacrylat (TMPTA; SR-351, Sartomer) und 10.00 g (13.9 mmol) Aldimin ***AL3*** auf 105 °C erwärmt und solange auf dieser Temperatur gehalten, bis die Acrylbande im FT-IR-Spektrum ($\delta_{C=C\text{-}H\ oop}$ bei 808 $cm^{-1}$) verschwunden war (18 Stunden). Man erhielt eine gelbe, dickflüssige, klare und geruchlose Flüssigkeit, die einen Amin-Gehalt von 3.48 mmol $NH_2$/g aufwies.
IR: 2952sh, 2922, 2851, 1736 (C=O), 1667 (C=N), 1465, 1418, 1392, 1374, 1347, 1300sh, 1246, 1163, 1113, 1054, 1057, 1017, 999, 935, 879, 781, 722.

**Beispiel 15 (Aldimin-haltige Verbindung *AV15*)**

In einem Rundkolben wurden unter Stickstoffatmosphäre eine Mischung von 9.56 g (52.0 mmol Epoxy) Bisphenol-A-diglycidylether (DGEBA oder BPADGE; Araldite® GY-250, Huntsman) und 20.00 g (52.0 mmol) Aldimin ***AL1*** auf 70 °C erwärmt und solange auf dieser Temperatur gehalten, bis die Epoxybanden im FT-IR-Spektrum ($\nu_{C\text{-}O\ asy}$ bei 914 und 861 $cm^{-1}$) verschwunden waren (16 Stunden). Man erhielt eine farblose, hochviskose, klare und geruchlose Flüssigkeit, die einen Amin-Gehalt von 3.50 mmol $NH_2$/g aufwies. IR: 3420(O-H),3034,2922,2851,2064,1884,1736(C=O),1667 (C=N), 1607, 1580, 1509, 1463,1417,1375,1297,1248,1180,1157,1108,1084,1038,933, 883, 827, 806, 767, 722.

Einkomponentige Kunststoffvorläufer

**Beispiele 16 bis 22 und Beispiel 23 (Vergleich)**

Für jedes Beispiel wurden 100.0 g Polyurethanpolymer ***PP1***, dessen Herstellung nachfolgend beschrieben wird, in einen Polypropylenbecher mit Schraubverschluss eingewogen und unter trockenen Stickstoff gestellt. Dazu wurden 0.3 g einer Salicylsäurelösung (5 Gewichts-% in Dioctyladipat) gegeben sowie das der Tabelle 1 aufgeführte Aldimin der Formel (XI) in der angegebenen Menge gegeben, die Mischung mittels eines Zentrifugalmischers (SpeedMixer™ DAC 150, FlackTek Inc.) innig vermengt, unmittelbar danach in eine innenlackierte Aluminiumtube abgefüllt und diese luftdicht verschlossen. Die Menge an zugegebenem Aldimin der Formel (XI) entspricht für alle Beispiele einem Verhältnis von 1.0 / 0.7 zwischen den Isocyanatgruppen im Polyurethanpolymer und der Summe der Reaktivgruppen (Aldiminogruppen plus Amino- oder Hydroxylgruppen) im Aldimin.

Das Polyurethanpolymer ***PP1*** wurde wie folgt hergestellt:

1300 g Polyoxypropylen-Diol (Acclaim® 4200 N, Bayer; OH-Zahl 28.5 mg KOH/g), 2600 g Polyoxypropylenpolyoxyethylen-Triol (Caradol® MD34-02, Shell; OH-Zahl 35.0 mg KOH/g), 605 g 4,4'-Methylendiphenyldiisocyanat (MDI; Desmodur® 44 MC L, Bayer) und 500 g Diisodecylphthalat (DIDP; Palatinol® Z, BASF) wurden bei 80 °C zu einem NCO-terminierten Polyurethanpolymeren mit einem titrimetrisch bestimmten Gehalt an freien Isocyanat-Gruppen von 2.07 Gewichts-% und einer Viskosität bei 20 °C von 48 Pa·s umgesetzt.

Der so erhaltene einkomponentige Kunststoffvorläufer wurde auf Lagerstabilität, Hautbildungszeit, Blasenbildung, Geruch und mechanische Eigenschaften nach Aushärtung geprüft.

Die **Lagerstabilität** wurde über die Veränderung der Viskosität während der Lagerung in der Wärme bestimmt. Dazu wurde der Kunststoffvorläufer in der verschlossenen Tube im Ofen bei 60 °C gelagert und seine Viskosität ein erstes Mal nach 12 Stunden, ein zweites Mal nach 7 Tagen Lagerdauer gemessen. Die Lagerstabilität ergibt sich aus der prozentualen Zunahme des zweiten Viskositätswerts gegenüber dem ersten.

Die Ergebnisse der Prüfungen sind in der Tabelle 1 aufgeführt.

Tabelle 1: Zusammensetzung und Lagerstabilität von einkomponentigen Kunststoffvorläufern.

| Beispiel | Aldimin der Formel (XI) | Aldiminzugabe [g] | $\frac{[NCO]}{[[OH]+[NH]]}$ | Viskositätszunahme [%][a] |
|---|---|---|---|---|
| **(Ref)**[b] | - | - | - | 16 |
| **16** | ***AL1*** | 6.6 | 1.0/0.7 | 18 |
| **17** | ***AL2*** | 7.9 | 1.0/0.7 | 26 |
| **18** | ***AL3*** | 8.3 | 1.0 / 0.7 | 18 |
| **19** | ***AL4*** | 9.4 | 1.0/0.7 | 25 |
| **20** | ***AL5*** | 8.5 | 1.0 / 0.7 | 27 |
| **21** | ***AL6*** | 7.8 | 1.0 / 0.7 | 13 |
| **22** | ***AL7*** | 11.2 | 1.0 / 0.7 | 23 |
| **23 (Vergleich)** | ***AL8*** | 4.9 | 1.0 / 0.7 | 42 |

[a] = (Viskosität nach 7 d / Viskosität nach 12 Std. - 1) × 100%.
[b] Referenzbeispiel ohne Aldimin.

Aus der Tabelle 1 ist ersichtlich, dass die einkomponentigen Kunststoffvorläufer der Beispiele 16 bis 22, welche erfindungsgemässe Aldimin-haltige Verbindungen der Formel (I) enthalten, die in situ aus dem Polyurethanpolymer ***PP1*** und den Aldiminen ***AL1*** bis ***AL7*** der Formel (XI) hergestellt wurden, eine gegenüber dem Kunststoffvorläufer des Referenzbeispiels, der keine Aldimin-haltige Verbindung enthält, eine vergleichbar grosse Viskositätserhöhung nach Lagerung aufweisen. Demgegenüber steigt die Viskosität des Kunststoffvorläufers des Vergleichsbeispiels 23, welcher eine Aldimin-haltige Verbindung nach dem Stand der Technik enthält, die in situ aus dem Polyurethanpolymer ***PP1*** und dem Aldimin ***AL8*** hergestellt wurde, deutlich stärker an.

Zur Bestimmung der **Hautbildungszeit** (Zeit bis zur Klebefreiheit, "tack-free time") wurde ein kleiner Teil des während 12 Stunden bei 60 ˚C gelagerten, raumtemperaturwarmen Kunststoffvorläufers in einer Schichtdicke von 3 mm auf Pappkarton aufgetragen und bei 23 ˚C und 50% relativer Luftfeuchtigkeit die Zeit bestimmt, die es dauerte, bis beim leichten Antippen der Kunststoffoberfläche mittels einer Pipette aus LDPE erstmals keine Rückstände auf der Pipette mehr zurückblieben.

Zur Bestimmung der **mechanischen Eigenschaften** nach Aushärtung wurde ein weiterer Teil des während 12 Stunden bei 60 ˚C gelagerten Kunststoffvorläufers als Film von ca. 2 mm Dicke in ein mit PTFE beschichtetes Blech gegossen, worauf man den Film während 7 Tagen bei 23 ˚C und 50% relativer Luftfeuchtigkeit zu einem elastischen Kunststoff aushärten liess. Der so hergestellte Kunststofffilm wurde gemäss DIN EN 53504 auf **Zugfestigkeit**, **Bruchdehnung** und **E-Modul** geprüft (Zuggeschwindigkeit: 200 mm/min). Qualitativ beurteilt wurden ausserdem die **Blasenbildung** (anhand der Menge Blasen, die während der Aushärtung des Films auftraten) sowie der Geruch (durch Riechen mit der Nase in einem Abstand von 10 cm, zuerst am frisch gegossenen Film und wieder am vollständig ausgehärteten Film).

Die Ergebnisse der Prüfungen sind in der Tabelle 2 aufgeführt.

Aus Tabelle 2 ist ersichtlich, dass die einkomponentigen Kunststoffvorläufer der Beispiele 16 bis 22, welche jeweils eine in situ hergestellte erfindungsgemässe Aldimin-haltige Verbindung der Formel (I) enthalten, rasch und ohne Blasenbildung aushärten, geruchsfrei sind und im ausgehärteten Zustand gute mechanische Eigenschaften besitzen. Demgegenüber härtet der Kunststoffvorläufer des Vergleichsbeispiels 23, welcher eine in situ hergestellte Aldimin-haltige Verbindung nach dem Stand der Technik enthält, langsamer und unter partieller Blasenbildung aus und weist einen starken Geruch auf.

Tabelle 2: Eigenschaften während und nach der Aushärtung von einkomponentigen Kunststoffvorläufern.

| Beispiel | **16** | **17** | **18** | **19** | **20** | **21** | **22** | **23 (Vgl)** |
|---|---|---|---|---|---|---|---|---|
| Hautbildung (min.) | 35 | 45 | 35 | 50 | 40 | 35 | 45 | 90 |
| Blasenbildung | keine | keine | keine | keine | keine | keine | keine | einige |
| Zugfestigkeit (MPa) | 0.8 | 1.0 | 0.8 | 0.7 | 0.7 | 0.7 | 1.0 | 1.0 |
| Bruchdehnung (%) | 180 | 200 | 60 | 70 | 80 | 130 | 220 | 240 |
| E-Modul (MPa)[a] | 1.3 | 1.4 | 2.3 | 1.7 | 1.6 | 1.3 | 1.3 | 1.1 |
| Geruch | kein | kein | kein | kein | kein | kein | kein | stark |
| [a] bei 0.5-5.0% Dehnung. | | | | | | | | |

**Beispiele 24 und 25**

Jeweils 50 g einer der in der Tabelle 3 aufgeführten Aldimin-haltigen Verbindungen der Formel (I) wurden in einen Polypropylenbecher mit Schraubverschluss eingewogen und unter trockenen Stickstoff gestellt. Dazu wurden 0.3 g einer Salicylsäurelösung (5 Gewichts-% in Dioctyladipat) gegeben, die Mischung mittels eines Zentrifugalmischers (SpeedMixer™ DAC 150, FlackTek Inc.) innig vermengt, unmittelbar danach in eine innenlackierte Aluminiumtube abgefüllt und diese luftdicht verschlossen.

Der so erhaltene einkomponentige Kunststoffvorläufer wurde auf Klebrigkeit sowie nach den für das Beispiel 16 beschriebenen Methoden auf Lagerstabilität, Hautbildungszeit, Blasenbildung, Geruch und mechanische Eigenschaften nach Aushärtung geprüft.

Die Prüfung auf **Klebrigkeit** erfolgte qualitativ durch Berühren mit dem Finger der Oberfläche des zur Bestimmung der mechanischen Eigenschaften angesetzten, während 1 Tag bei 23 ˚C und 50% relativer Luftfeuchtigkeit partiell ausgehärteten Kunststofffilms.

Die Ergebnisse der Prüfungen sind in der Tabelle 3 aufgeführt.

Tabelle 3: Lagerstabilität, Eigenschaften während und nach der Aushärtung Eigenschaften während und nach der Aushärtung von einkomponentigen Kunststoffvorläufern.

| Beispiel | **24** | **25** |
|---|---|---|
| Aldimin-haltige Verbindung | ***AV6*** | ***AV7*** |
| Viskositätszunahme in % | 5 | 2 |
| Hautbildungszeit (min.) | 26 | 40 |

(fortgesetzt)

| Beispiel | **24** | **25** |
|---|---|---|
| Blasenbildung | keine | keine |
| Klebrigkeit | schwach | keine |
| Zugfestigkeit (MPa) | 1.0 | 1.0 |
| Bruchdehnung (%) | 2900 | 100 |
| E-Modul (MPa)[a] | 0.9 | 1.8 |
| Geruch | kein | kein |
| [a] bei 0.5-5.0% Dehnung. | | |

Aus der Tabelle 3 ist ersichtlich, dass die einkomponentigen Kunststoffvorläufer der Beispiele 24 und 25, welche jeweils eine Aldimin-haltige Verbindung der Formel (I) ***AV6*** und ***AV7*** enthalten, bei der Lagerung nur einen geringfügigen Viskositätsanstieg zeigen. Sie härten bei ihrer Anwendung rasch und ohne Blasenbildung aus, sind geruchsfrei und verfügen im ausgehärteten Zustand über gute mechanische Eigenschaften.

**Beispiele 26 und 27**

Jeweils eine der in der Tabelle 4 aufgeführten Aldimin-haltigen Verbindungen der Formel (I) wurden zusammen mit dem Polyurethanpolymer ***PP2,*** dessen Herstellung nachfolgend beschrieben wird, in den angegebenen Mengen in einen Polypropylenbecher mit Schraubverschluss eingewogen und unter trockenen Stickstoff gestellt. Dazu wurden 0.3 g einer Salicylsäurelösung (5 Gewichts-% in Dioctyladipat) gegeben, die Mischung mittels eines Zentrifugalmischers (SpeedMixer™ DAC 150, FlackTek Inc.) innig vermengt, unmittelbar danach in eine innenlackierte Aluminiumtube abgefüllt und diese luftdicht verschlossen. Die Menge an zugegebener Verbindung der Formel (I) entspricht einem Verhältnis von 1 / 1 zwischen den Isocyanatgruppen im Polyurethanpolymer und den Aldiminogruppen in der Verbindung der Formel (I).

Das Polyurethanpolymer ***PP2*** wurde wie folgt hergestellt:

3960 g Polyoxypropylen-Diol (Acclaim® 4200 N, Bayer; OH-Zahl 28.5 mg KOH/g), 540 g 4,4'-Methylendiphenyldiisocyanat (MDI; Desmodur® 44 MC L, Bayer) und 500 g Diisodecylphthalat (DIDP; Palatinol® Z, BASF) wurden bei 80 ˚C zu einem NCO-terminierten Polyurethanpolymeren mit einem titrimetrisch bestimmten Gehalt an freien Isocyanat-Gruppen von 1.86 Gewichts-% und einer Viskosität bei 20 ˚C von 24 Pa·s umgesetzt.

Tabelle 4: Zusammensetzung der einkomponentigen Kunststoffvorläufer der Beispiele 26 und 27.

| Beispiel | Polyurethanpolymer **PP2** | Aldimin-haltige Verbindung |
|---|---|---|
| **26** | 23.04 g | ***AV8***, 26.96 g |
| **27** | 30.13 g | ***AV9***, 19.87 g |

Die so erhaltenen einkomponentigen Kunststoffvorläufer wurden nach den für das Beispiel 16 beschriebenen Methoden auf Lagerstabilität, Hautbildungszeit, Blasenbildung, Geruch und mechanische Eigenschaften nach Aushärtung geprüft sowie nach der für das Beispiel 24 beschriebenen Methode nach Klebrigkeit.Die Ergebnisse der Prüfungen sind in Tabelle 5 aufgeführt.

Tabelle 5: Lagerstabilität, Eigenschaften während und nach der Aushärtung Eigenschaften während und nach der Aushärtung von einkomponentigen Kunststoffvorläufern.

| Beispiel | **26** | **27** |
|---|---|---|
| Viskositätszunahme in % | 6 | 13 |
| Hautbildungszeit (min.) | 16 | 18 |
| Blasenbildung | keine | keine |
| Klebrigkeit | keine | keine |
| Zugfestigkeit (MPa) | 3.5 | 1.0 |

(fortgesetzt)

| Beispiel | 26 | 27 |
|---|---|---|
| Bruchdehnung (%) | 2100 | 90 |
| E-Modul (MPa)[a] | 1.1 | 1.9 |
| Geruch | kein | kein |
| [a] bei 0.5-5.0% Dehnung. | | |

Aus Tabelle 5 ist ersichtlich, dass die einkomponentigen Kunststoffvorläufer der Beispiele 26 und 27, welche jeweils eine Aldimin-haltige Verbindung der Formel (I) ***AV8*** und ***AV9*** enthalten, bei der Lagerung nur einen geringfügigen Viskositätsanstieg zeigen. Sie härten bei ihrer Anwendung rasch und ohne Blasenbildung aus, sind geruchsfrei und verfügen im ausgehärteten Zustand über gute mechanische Eigenschaften.

Als Klebstoffe verwendbare einkomponentige Kunststoffvorläufer

**Beispiele 28 bis 35 und Beispiel 36 (Vergleich)**

Für jedes der Beispiele wurden die in der Tabelle 6 aufgeführten Substanzen in der angegebenen Menge (in Gewichtsteilen) in einem Vakuummischer unter Feuchtigkeitsausschluss zu einer knollenfreien, homogenen Paste verarbeitet, diese unverzüglich in eine innenlackierte Aluminium-Kartusche abgefüllt und die Kartusche luftdicht verschlossen. Die Polyurethanpolymere ***PP1*** und ***PP2*** wurden wie bei Beispiel 16, bzw. Beispiel 26, beschrieben hergestellt.

Das Polyaldimin ***PA1*** wurde hergestellt aus der Kondensationsreaktion zwischen 1,6-Hexamethylendiamin und 2,2-Dimethyl-3-lauroyloxy-propanal (im Molverhältnis zwischen Amino- und Aldehyd-Gruppen von 1 zu 1.05) und hatte einen Amin-Gehalt von 2.94 mmol $NH_2$/g.

Das Polyaldimin ***PA2*** wurde hergestellt aus der Kondensationsreaktion zwischen alpha, omega-Polyoxypropylendiamin (Jeffamine® D-230, Huntsman; Amin-Gehalt = 8.29 mmol $NH_2$/g) und 2,2-Dimethyl-3-lauroyloxy-propanal (im Molverhältnis zwischen Amino- und Aldehyd-Gruppen von 1 : 1.05) und hatte einen Amin-Gehalt von 2.50 mmol $NH_2$/g.

Das Verhältnis zwischen den Isocyanatgruppen im Polyurethanpolymer und der Summe der Reaktivgruppen (Aldiminogruppen plus Amino- und Hydroxylgruppen) in den Aldimin-haltigen Verbindungen der Formel (I), den Aldiminen der Formel (XI) und den Polyaldiminen beträgt für alle Beispiele 1.0 / 0.7.

Die so erhaltenen Klebstoffe wurden auf Hautbildungszeit, Geruch, mechanische Eigenschaften nach Aushärtung und auf Haftungseigenschaften auf Glas geprüft. Die Ergebnisse der Prüfungen sind in der Tabelle 7 aufgeführt.

Die **Shore A**-Härte wurde bestimmt nach DIN 53505.

Tabelle 6: Zusammensetzung von Klebstoffen.

| Beispiel | 28 | 29 | 30 | 31 | 32 | 33 | 34 | 35 | 36 (vgl) |
|---|---|---|---|---|---|---|---|---|---|
| Polyurethanpolymer | ***PP1***, 50.0 | ***PP1***, 50.0 | ***PP1***, 50.0 | ***PP2,*** 50.0 | ***PP2,*** 50.0 | ***PP2,*** 50.0 | ***PP2***, 50.0 | ***PP2,*** 50.0 | ***PP1***, 50.0 |
| Aldimin(e) der Formel (XI) | ***AL1***, 3.3 | ***AL2***, 3.9 | ***AL6***, 3.9 | ***AL1***, 1.8; ***AL3***, 1.9 | ***AL4***, 1.9 | - | - | - | ***AL8***, 2.5 |
| Aldimin-halt.Verb. der Formel (I)[a] | - | - | - | - | - | ***AV1***, 2.6 | ***AV2***, 3.0 | ***AV3***, 2.5 | - |
| Polyaldimin | - | - | - | - | ***PA1***, 2.9 | ***PA2***, 3.7 | ***PA2***, 2.5 | ***PA1***, 2.9 | - |
| Weichmacher[b] | 12.2 | 11.6 | 11.6 | 11.8 | 10.7 | 9.9 | 10.6 | 10.7 | 13.0 |
| Kaolin | 17.0 | 17.0 | 17.0 | 17.0 | 17.0 | 17.0 | 17.0 | 17.0 | 17.0 |
| Russ | 17.0 | 17.0 | 17.0 | 17.0 | 17.0 | 17.0 | 17.0 | 17.0 | 17.0 |
| Trocknungsmittel[c] | 0.1 | 0.1 | 0.1 | 0.1 | 0.1 | 0.1 | 0.1 | 0.1 | 0.1 |
| Epoxysilan[d] | 0.3 | 0.3 | 0.3 | 0.3 | 0.3 | 0.3 | 0.3 | 0.3 | 0.3 |

(fortgesetzt)

| Beispiel | **28** | **29** | **30** | **31** | **32** | **33** | **34** | **35** | **36 (vgl)** |
|---|---|---|---|---|---|---|---|---|---|
| Katalysator[e] | 0.1 | 0.1 | 0.1 | 0.1 | 0.1 | 0.1 | 0.1 | 0.1 | 0.1 |
| [a] Aldimin-haltige Verbindung der Formel (I). [b] Diisodecylphthalat (DIDP; Palatinol® Z, BASF). [c] p-Tosylisocyanat. [d] 3-Glycidoxypropyltrimethoxysilan (Silquest® A-187, OSi Crompton). [e] Salicylsäure (5 Gewichts-% in Dioctyladipat). | | | | | | | | | |

Zur Prüfung der **Zugscherfestigkeit** wurden für jede Messung jeweils 2 Glasplättchen von 6 mm Dicke, 25 mm Breite und 75 mm Länge (Floatglas; Firma Rocholl, Schönbrunn, Deutschland) mit Sika® Aktivator (erhältlich bei Sika Schweiz AG) vorbehandelt. Nach einer Ablüftezeit von 10 Minuten wurden die Plättchen mit Hilfe einer passenden PTFE-Form im vertikalen Abstand von 5 mm so gegeneinander angeordnet, dass sie an den Kopfenden um 10 mm überlappten. Der Überlappungsbereich zwischen den Plättchen wurde mit Klebstoff befüllt, wobei dieser auf die aktivierten Seiten der Plättchen zu liegen kam. Der Klebstoff wurde während 7 Tagen bei 23 °C und 50% relativer Luftfeuchtigkeit ausgehärtet und dann wurde mit Hilfe einer Zugprüfmaschine die Bruchkraft bei einer konstanten Querjochgeschwindigkeit von 20 mm/min nach DIN EN 1465 bestimmt. Die angegebenen Werte sind Mittelwerte aus drei Messungen.

Die **Haftung auf Glas** wurde anhand des Bruchbildes auf den für die Bestimmung der Zugscherfestigkeit verwendeten Prüfkörper nach erfolgter Prüfung ermittelt. Ein 100% kohäsiver, das heisst vollständig im Klebstoff verlaufender Bruch wurde mit dem Wert "1" bewertet, während ein 0% kohäsiver, das heisst vollständig zwischen Glasoberfläche und Klebstoff verlaufender und damit adhäsiver Bruch mit dem Wert "5" bewertet wurde. Haftungen mit Kohäsionsbruchwerten von weniger als 75% gelten als ungenügend.
Die übrigen Prüfungen wurden wie bei Beispiel 16 beschrieben durchgeführt.

Tabelle 7: Eigenschaften während und nach der Aushärtung der Klebstoffe Zusammensetzung von Klebstoffen.

| Beispiel | **28** | **29** | **30** | **31** | **32** | **33** | **34** | **35** | **36 (Vgl)** |
|---|---|---|---|---|---|---|---|---|---|
| Hautbildungszeit[a] | 23 | 27 | 18 | 40 | 60 | 68 | 80 | 50 | 90 |
| Geruch | kein | kein | kein | kein | kein | kein | kein | kein | stark |
| Shore A-Härte | 53 | 60 | 58 | 54 | 65 | 56 | 56 | 66 | 56 |
| Zugfestigkeit (MPa) | 9.7 | 9.9 | 9.9 | 10.2 | 9.1 | 8.1 | 9.0 | 7.0 | 9.6 |
| Bruchdehnung (%) | 650 | 590 | 530 | 720 | 530 | 700 | 650 | 450 | 550 |
| E-Modul (MPa)[b] | 3.6 | 4.6 | 4.0 | 3.3 | 7.3 | 4.1 | 4.0 | 9.0 | 4.0 |
| Zugscherfestigkeit[c] | 8.6 | 7.5 | 10.1 | 9.9 | 8.5 | 6.3 | 6.7 | 6.4 | 3.9 |
| Haftung auf Glas | 1 | 1 | 1 | 1 | 1 | 1 | 1 | 1 | 5 |
| [a] in Minuten. [b] bei 0.5-5.0% Dehnung. [c] in MPa. | | | | | | | | | |

Aus der Tabelle 7 ist ersichtlich, dass die Klebstoffe der Beispiele 28 bis 35, welche Aldimin-haltige Verbindungen der Formel (I) enthalten, die in den Beispielen 28 bis 32 im Zuge der Klebstoffherstellung (in situ) aus Aldiminen der Formel (XI) und den Polyurethanpolymeren ***PP1*** oder ***PP2*** hergestellt wurden und in den Beispielen 33 bis 35 vorgängig hergestellt und, zum Teil kombiniert mit Polyaldiminen, in den Klebstoff eingemischt wurden, rasch aushärten, geruchsfrei sind und im ausgehärteten Zustand sehr gute mechanische Eigenschaften besitzen. Demgegenüber härtet der Klebstoff des Vergleichsbeispiels 36, welcher eine Aldimin-haltige Verbindung nach dem Stand der Technik, die in situ aus dem Aldimin ***AL8*** und dem Polyurethanpolymer ***PP1*** hergestellt wurde, enthält, langsamer aus, weist einen starken Geruch auf und verfügt über eine ungenügende Haftung zu Glas.

## Patentansprüche

1. Aldimin-haltige Verbindung der Formel (I)

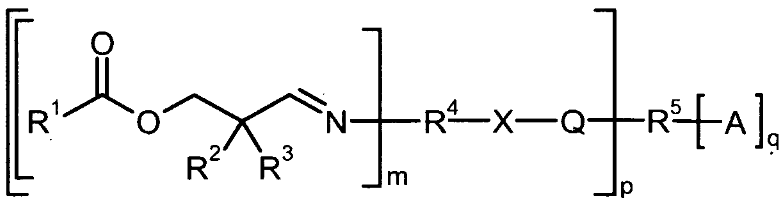

(I)

wobei
m für eine ganze Zahl von 1 bis 4 steht,
p für eine ganze Zahl von 1 bis 6 steht und
q für eine ganze Zahl von 0 bis 5 steht
mit der Massgabe, dass p+q = 2 bis 6 ist;
und wobei
$R^1$ entweder
für einen einwertigen Kohlenwasserstoffrest mit 6 bis 30 C-Atomen, welcher gegebenenfalls mindestens ein Heteroatom, insbesondere in Form von Ether-Sauerstoff, aufweist, steht,
oder
für einen Substituenten der Formel (II) steht

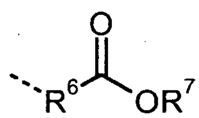

(II)

wobei
$R^6$ für einen zweiwertigen Kohlenwasserstoffrest mit 2 bis 20 C-Atomen, welcher gegebenenfalls mindestens ein Heteroatom, insbesondere in Form von Ether-Sauerstoff, aufweist, steht,
und
$R^7$ für einen einwertigen Kohlenwasserstoffrest mit 1 bis 20 C-Atomen steht;
und wobei
$R^2$ und $R^3$ entweder,
unabhängig voneinander, je für einen einwertigen Kohlenwasserstoffrest mit 1 bis 12 C-Atomen stehen;
oder
zusammen einen zweiwertigen Kohlenwasserstoffrest mit 4 bis 20 C-Atomen bilden, der Teil eines, gegebenenfalls substituierten, carbocyclischen Rings mit 5 bis 8, bevorzugt 6, C-Atomen ist;
und wobei
$R^4$ für einen (m+1)-wertigen Kohlenwasserstoffrest mit 2 bis 12 C-Atomen, welcher gegebenenfalls mindestens ein Heteroatom, insbesondere in Form von Ether-Sauerstoff oder tertiärem AminStickstoff, enthält, steht;
und wobei
X für O, S oder N-$R^8$ steht,
wobei
$R^8$ entweder
für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureestergruppe aufweist, steht,
oder
für einen Substituenten der Formel (III) steht

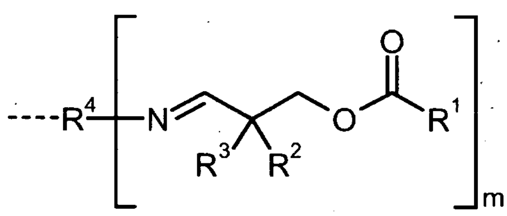

(III),

und wobei
A für eine Reaktivgruppe steht,ausgewählt aus der Gruppe umfassend
---N=C=$Y^1$

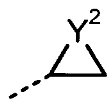 , 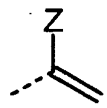 ,

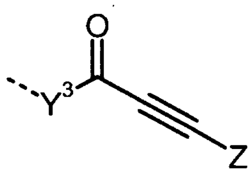 , 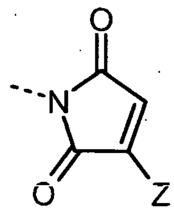 und 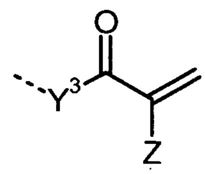

wobei
$Y^1$ für O oder S steht, $Y^2$ für O, S oder N-H steht, Z für H oder $CH_3$ und $Y^3$ für O oder N-H steht;
und
$R^5$ entweder
für einen (p+q)-wertigen organischen, gegebenenfalls Heteroatome enthaltenden Rest, wie er durch Entfernung von p+q Resten A aus $R^5$-$[A]_{p+q}$ erhalten wird, steht
oder
für N, $NR^{14}$, O, OC(O)O, Si, $P(O)O_3$ oder $SO_2$ steht,
wobei
$R^{14}$ für einen einwertigen Kohlenwasserstoffrest mit 1 bis 20 C-Atomen steht;
und
Q für einen Substituenten steht, welcher ausgewählt ist aus der Gruppe umfassend

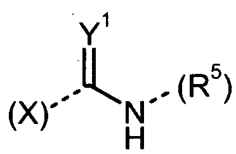 , 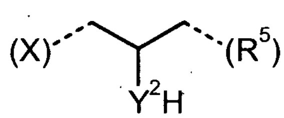 , 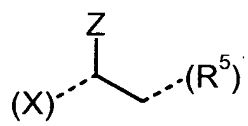 ,

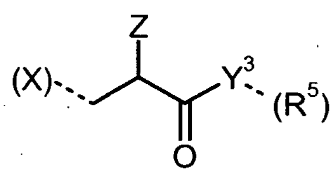 , 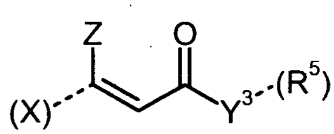 und 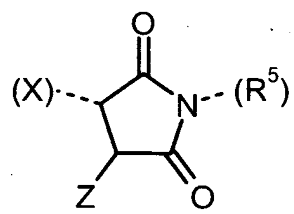

wobei die gestrichelten Linien die Bindungen zu den angebenen Substituenten darstellen.

2. Aldimin-haltige Verbindung gemäss Anspruch 1, **dadurch gekennzeichnet, dass** $R^2$ und $R^3$ gleich sind und insbesondere je für eine Methylgruppe stehen.

3. Aldimin-haltige Verbindung gemäss Anspruch 1 oder 2, **dadurch gekennzeichnet, dass** m = 2 oder 1, insbesondere 1, ist.

4. Aldimin-haltige Verbindung gemäss einem der vorhergehenden Ansprüche, **dadurch gekennzeichnet, dass** X für N-$R^8$ steht und $R^8$ für einen einwertigen Kohlenwasserstoffrest der Formel (IX) oder (IX') steht

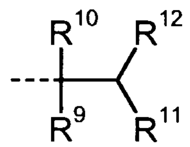 (IX)

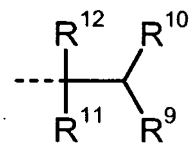 (IX')

wobei $R^9$ für einen Rest, welcher ausgewählt ist aus der Gruppe bestehend aus $-COOR^{13}$, -CN, $-NO_2$, $-PO(OR^{13})_2$, $-SO_2R^{13}$ und $-SO_2OR^{13}$, steht; $R^{10}$ für ein Wasserstoffatom oder einen Rest ausgewählt aus der Gruppe bestehend aus $-R^{13}$, $-COOR^{13}$ und $-CH_2COOR^{13}$ steht und

$R^{11}$ und $R^{12}$ unabhängig voneinander für ein Wasserstoffatom oder einen Rest ausgewählt aus der Gruppe bestehend aus $-R^{13}$, $-COOR^{13}$ und -CN, wobei $R^{13}$ für einen einwertigen Kohlenwasserstoffrest mit 1 bis 20 C-Atomen steht.

5. Aldimin-haltige Verbindung gemäss einem der vorhergehenden Ansprüche, **dadurch gekennzeichnet, dass** X für O oder S steht.

6. Aldimin-haltige Verbindung gemäss einem der vorhergehenden Ansprüche, **dadurch gekennzeichnet, dass** diese durch die Umsetzung eines Aldimins der Formel (XI) mit einer Verbindung D der Formel (XII) mit den Reaktivgruppen A und A' hergestellt wird

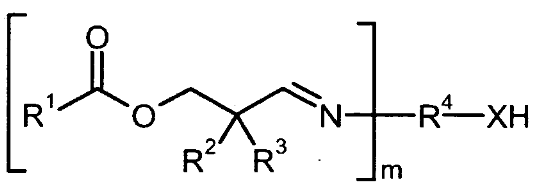 (XI)

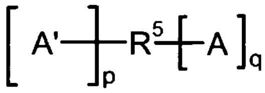 (XII)

wobei A' für eine Reaktivgruppe steht, welcher ausgewählt ist aus der Gruppe umfassend

$----N=C=Y^1$

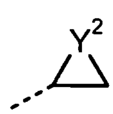 , 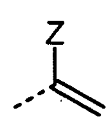 ,

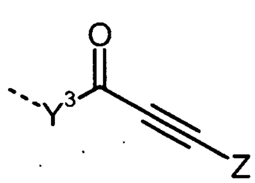 , 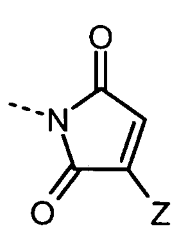 und 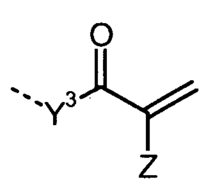

und wobei A' identisch mit der Reaktivgruppe A oder davon verschieden sein kann.

7. Aldimin-haltige Verbindung gemäss einem der vorhergehenden Ansprüche, **dadurch gekennzeichnet, dass** die Verbindung **D** ein Polyisocyanat ist.

8. Aldimin-haltige Verbindung gemäss Anspruch 6, **dadurch gekennzeichnet, dass** die Verbindung **D** ein Polyepoxid ist.

9. Aldimin-haltige Verbindung gemäss Anspruch 6, **dadurch gekennzeichnet, dass** die Verbindung **D** ein Poly(meth) acrylat ist.

10. Aldimin-haltige Verbindung gemäss einem der Ansprüche 6 - 9, **dadurch gekennzeichnet, dass** das Aldimin der Formel (XI) in einem Verhältnis von einem Molequivalent aktiven Wasserstoff des Aldimins auf ein Molequivalent Reaktivgruppen der Verbindung **D** eingesetzt wird.

11. Aldimin-haltige Verbindung gemäss einem der Ansprüche 6 - 9, **dadurch gekennzeichnet, dass** das Aldimin der Formel (XI) in einem Verhältnis von weniger als einem Molequivalent aktiven Wasserstoff des Aldimins auf ein Molequivalent Reaktivgruppen der Verbindung **D** eingesetzt wird.

12. Aldimin-haltige Verbindung gemäss einem der Ansprüche 6 - 11, **dadurch gekennzeichnet, dass** die Reaktivgruppe A verschieden von der Reaktivgruppe A' ist.

13. Verwendung einer Aldimin-haltigen Verbindung (I) gemäss einem der Ansprüche 1 bis 12 als Quelle eines Aldehyds der Formel (IV)

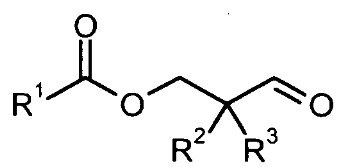
(IV)

14. Verfahren der Hydrolyse einer Aldimin-haltigen Verbindung gemäss einem der Ansprüche 1 bis 12.

15. Verfahren zur Vernetzung einer Aldimin-haltigen Verbindung gemäss einem der Ansprüche 1 bis 12, wobei das Produkt aus m und p der Formel (I) einen Wert von q oder kleiner ergibt, unter dem Einfluss von Wasser.

16. Zusammensetzung enthaltend einer Aldimin-haltigen Verbindung gemäss einem der Ansprüche 1 bis 12.

## Claims

1. An aldimine-containing compound of the formula (I)

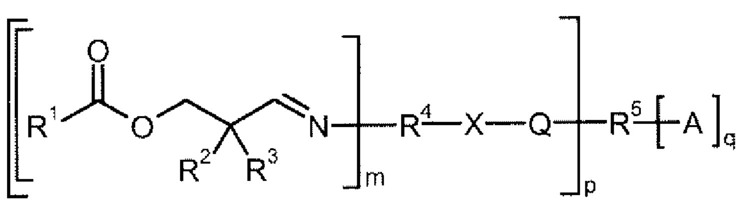
(I)

in which
m is an integer from 1 to 4,
p is an integer from 1 to 6 and
q is an integer from 0 to 5,
with the proviso that p+q is from 2 to 6;
and in which
$R^1$ either
is a monovalent hydrocarbon radical having 6 to 30 C atoms which optionally has at least one heteroatom, in particular in the form of ether oxygen,

or
is a substituent of the formula (II)

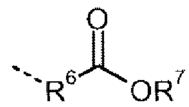 (II)

in which
$R^6$ is a divalent hydrocarbon radical having 2 to 20 C atoms which optionally has at least one heteroatom, in particular in the form of ether oxygen,
and
$R^7$ is a monovalent hydrocarbon radical having 1 to 20 C atoms;
and in which
$R^2$ and $R^3$ either,
independently of one another, are each a monovalent hydrocarbon radical having 1 to 12 C atoms;
or
together form a divalent hydrocarbon radical having 4 to 20 C atoms which is part of an optionally substituted, carbocyclic ring having 5 to 8, preferably 6, C atoms;
and in which
$R^4$ is an (m+1)-valent hydrocarbon radical which has 2 to 12 C atoms and optionally contains at least one heteroatom, in particular in the form of ether oxygen or tertiary amine nitrogen;
and in which
X is O, S or N-$R^8$,
in which
$R^8$ either
is a monovalent hydrocarbon radical which has 1 to 20 C atoms and optionally has at least one carboxylic acid ester, nitrile, nitro, phosphonic acid ester, sulfone or sulfonic acid ester group,
or
is a substituent of the formula (III)

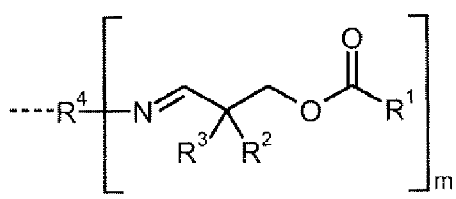 (III),

and in which
A is a reactive group selected from the group consisting of
$----N=C=Y^1$

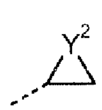 , 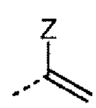 ,

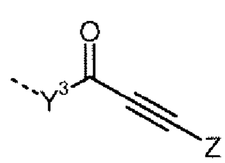 , 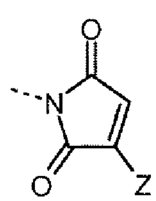 and 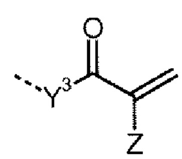 .

in which
$Y^1$ is O or S, $Y^2$ is 0, S or N-H, Z is H or $CH_3$ and $Y^3$ is O or N-H;
and
$R^5$ either
is a (p+q)-valent organic radical optionally containing heteroatoms, as obtained by removing p+q radicals A from $R^5$-$[A]_{p+q,}$
or
is N, $NR^{14}$, 0, OC(O)O, Si, $P(O)O_3$ or $SO_2$,
in which
$R^{14}$ is a monovalent hydrocarbon radical having 1 to 20 C atoms;
and
Q is a substituent which is selected from the group consisting of

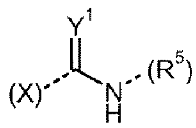 , 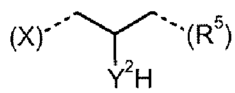 , 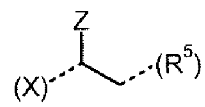

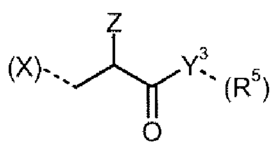 , 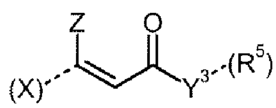 and 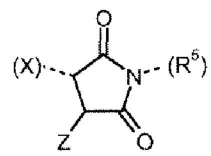

in which the dashed lines are the bonds to the stated substituents.

2. The aldimine-containing compound as claimed in claim 1, **characterized in that** $R^2$ and $R^3$ are identical and are in particular each a methyl group.

3. The aldimine-containing compound as claimed in claim 1 or 2, **characterized in that** m is 2 or 1, in particular 1.

4. The aldimine-containing compound as claimed in any of the preceding claims, **characterized in that** X is N-$R^8$ and $R^8$ is a monovalent hydrocarbon radical of the formula (IX) or (IX')

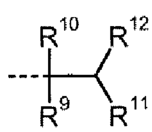 (IX) 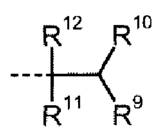 (IX')

in which $R^9$ is a radical which is selected from the group consisting of $-COOR^{13}$, -CN, $-NO_2$, $-PO(OR^{13})_2$, $-SO_2R^{13}$ and $-SO_2OR^{13}$;
$R^{10}$ is a hydrogen atom or a radical selected from the group consisting of $-R^{13}$, $-COOR^{13}$ and $-CH_2COOR^{13}$ and $R^{11}$ and $R^{12}$, independently of one another, are a hydrogen atom or a radical selected from the group consisting of $-R^{13}$, $-COOR^{13}$ and -CN, in which $R^{13}$ is a monovalent hydrocarbon radical having 1 to 20 C atoms.

5. The aldimine-containing compound as claimed in any of the preceding claims, **characterized in that** X is 0 or S.

6. The aldimine-containing compound as claimed in any of the preceding claims, **characterized in that** it is prepared by reacting an aldimine of the formula (XI) with a compound **D** of the formula (XII) having the reactive groups A and A'

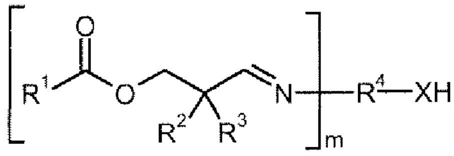

(XI)

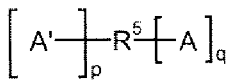

(XII)

in which A' is a reactive group which is selected from the group consisting of

----N=C=$Y^1$

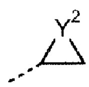 , 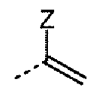 ,

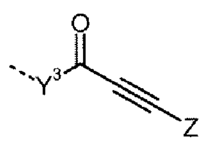 , 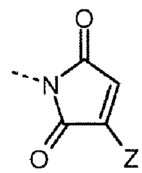 and 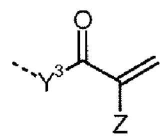 .

and in which A' may be identical to the reactive group A or different therefrom.

**7.** The aldimine-containing compound as claimed in any of the preceding claims, **characterized in that** the compound **D** is a polyisocyanate.

**8.** The aldimine-containing compound as claimed in claim 6, **characterized in that** the compound **D** is a polyepoxide.

**9.** The aldimine-containing compound as claimed in claim 6, **characterized in that** the compound **D** is a poly(meth) acrylate.

**10.** The aldimine-containing compound as claimed in any of claims 6 - 9, **characterized in that** the aldimine of the formula (XI) is used in a ratio of one mole equivalent of active hydrogen of the aldimine to one mole equivalent of reactive groups of the compound **D**.

**11.** The aldimine-containing compound as claimed in any of claims 6 - 9, **characterized in that** the aldimine of the formula (XI) is used in a ratio of less than one mole equivalent of active hydrogen of the aldimine to one mole equivalent of reactive groups of the compound **D**.

**12.** The aldimine-containing compound as claimed in any of claims 6 - 11, **characterized in that** the reactive group A is different from the reactive group A'.

**13.** The use of an aldimine-containing compound (I) as claimed in any of claims 1 to 12 as a source of an aldehyde of the formula (IV)

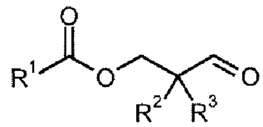 (IV)

14. A process for the hydrolysis of an aldimine-containing compound as claimed in any of claims 1 to 12.

15. A process for the crosslinking of an aldimine-containing compound as claimed in any of claims 1 to 12, wherein the product of m and p of the formula (I) gives a value of q or less, under the influence of water.

16. A composition containing an aldimine-containing compound as claimed in any of claims 1 to 12.

**Revendications**

1. Composé à fonction(s) aldimine de formule (I)

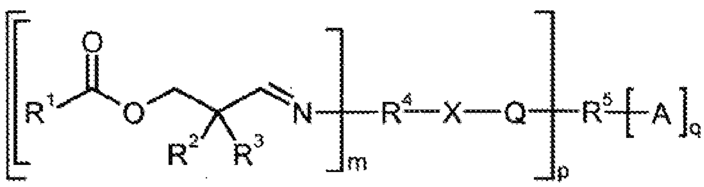 (I)

où

m vaut un nombre entier de 1 à 4,
p vaut un nombre entier de 1 à 6, et
q vaut un nombre entier de 0 à 5,

à condition que p + q = 2 à 6 ;
et où

$R^1$ représente
soit un radical hydrocarboné monovalent comprenant 6 à 30 atomes de carbone, qui présente le cas échéant au moins un hétéroatome, en particulier sous forme d'oxygène de fonction éther, soit un substituant de formule (II),

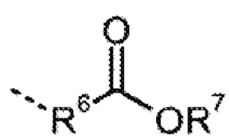 (II)

où

$R^6$ représente un radical hydrocarboné divalent comprenant 2 à 20 atomes de carbone, qui présente le cas échéant au moins un hétéroatome, en particulier sous forme d'oxygène de fonction éther, et
$R^7$ représente un radical hydrocarboné monovalent comprenant 1 à 20 atomes de carbone ;

et où

$R^2$ et $R^3$ soit représentent, indépendamment l'un de l'autre, à chaque fois un radical hydrocarboné monovalent

comprenant 1 à 12 atomes de carbone ; soit forment ensemble un radical hydrocarboné divalent comprenant 4 à 20 atomes de carbone, qui fait partie d'un cycle carbocyclique, le cas échéant substitué, comprenant 5 à 8, de préférence 6, atomes de carbone ;

et où

$R^4$ représente un radical hydrocarboné (m + 1)-valent comprenant 2 à 12 atomes de carbone, qui contient le cas échéant au moins un hétéroatome, en particulier sous forme d'oxygène de fonction éther ou d'azote de fonction amine tertiaire ;

et où

X représente O, S ou N-$R^8$,

où

$R^8$ représente
soit un radical hydrocarboné monovalent comprenant 1 à 20 atomes de carbone, qui présente le cas échéant au moins un groupe ester d'acide carboxylique, nitrile, nitro, ester d'acide phosphonique, sulfone ou ester d'acide sulfonique, soit un substituant de formule (III),

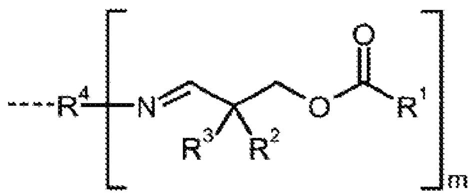 (III),

et où

A représente un groupe réactif, choisi dans le groupe comprenant ----N=C=$Y^1$

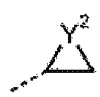 , 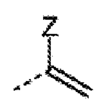 ,

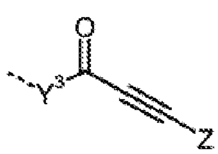 , 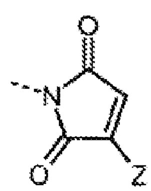 et 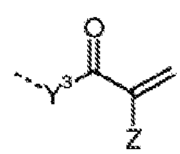

où

$Y^1$ représente O ou S,
$Y^2$ représente O, S ou N-H,
Z représente H ou $CH_3$ et
$Y^3$ représente O ou N-H;

et

$R^5$ représente
soit un radical organique, (p + q)-valent, contenant le cas échéant des hétéroatomes, tel qu'il est obtenu par élimination de p + q radicaux A à partir de $R^5$-$[A]_{p+q}$, soit représente N, $NR^{14}$, O, OC(O)O, Si, $P(O)O_3$ ou $SO_2$, où

$R^{14}$ représente un radical hydrocarboné monovalent comprenant 1 à 20 atomes de carbone ;

et

Q représente un substituant qui est choisi dans le groupe comprenant

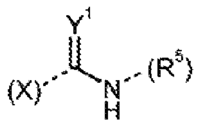

,

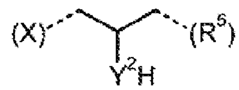

,

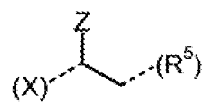

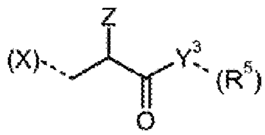

,

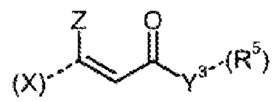

et

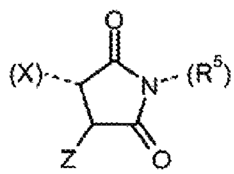

où les lignes en pointillés représentent les liaisons avec les substituants indiqués.

**2.** Composé à fonction(s) aldimine selon la revendication 1, **caractérisé en ce que** $R^2$ et $R^3$ sont identiques et représentent en particulier chacun un groupe méthyle.

**3.** Composé à fonction(s) aldimine selon la revendication 1 ou 2, **caractérisé en ce que** m = 2 ou 1, en particulier 1.

**4.** Composé à fonction(s) aldimine selon l'une quelconque des revendications précédentes, **caractérisé en ce que** X représente N-$R^8$ et $R^8$ représente un radical hydrocarboné monovalent de formule (IX) ou (IX')

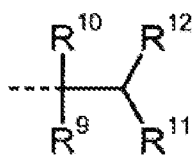

(IX)

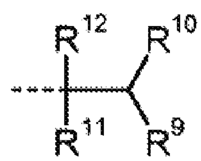

(IX')

où

$R^9$ représente un radical choisi dans le groupe constitué par $-COOR^{13}$, -CN, $-NO_2$, $-PO(OR^{13})_2$, $-SO_2R^{13}$ et $-SO_2OR^{13}$;
$R^{10}$ représente un atome d'hydrogène ou un radical choisi dans le groupe constitué par $-R^{13}$, $-COOR^{13}$ et $-CH_2COOR^{13}$ et
$R^{11}$ et $R^{12}$ représentent, indépendamment l'un de l'autre, un atome d'hydrogène ou un radical choisi dans le groupe constitué par $-R^{13}$, $-COOR^{13}$ et -CN, où $R^{13}$ représente un radical hydrocarboné monovalent comprenant 1 à 20 atomes de carbone.

**5.** Composé à fonction(s) aldimine selon l'une quelconque des revendications précédentes, **caractérisé en ce que** X représente O ou S.

**6.** Composé à fonction(s) aldimine selon l'une quelconque des revendications précédentes, **caractérisé en ce qu'**il est préparé par transformation d'une aldimine de formule (XI) avec un composé D de formule (XII) présentant les groupes réactifs A et A'

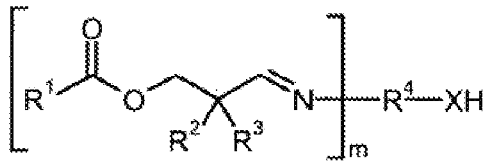

(XI)

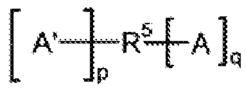

(XII)

où A' représente un groupe réactif, qui est choisi dans le groupe comprenant
----N=C=$Y^1$

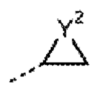 , 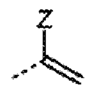 ,

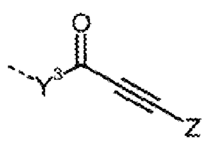 , 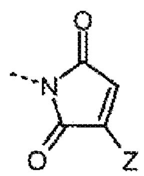 et 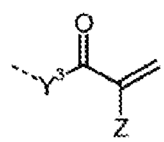

et où A' peut être identique au groupe réactif A ou différent de celui-ci.

**7.** Composé à fonction(s) aldimine selon l'une quelconque des revendications précédentes, **caractérisé en ce que** le composé **D** est un polyisocyanate.

**8.** Composé à fonction(s) aldimine selon la revendication 6, **caractérisé en ce que** le composé **D** est un polyépoxyde.

**9.** Composé à fonction(s) aldimine selon la revendication 6, **caractérisé en ce que** le composé **D** est un poly(méth) acrylate.

**10.** Composé à fonction(s) aldimine selon l'une quelconque des revendications 6-9, **caractérisé en ce que** l'aldimine de formule (XI) est utilisée dans un rapport d'un équivalent en mole d'hydrogène actif de l'aldimine pour un équivalent en mole de groupes réactifs du composé **D**.

**11.** Composé à fonction(s) aldimine selon l'une quelconque des revendications 6-9, **caractérisé en ce que** l'aldimine de formule (XI) est utilisée dans un rapport inférieur à un équivalent en mole d'hydrogène actif de l'aldimine pour un équivalent en mole de groupes réactifs du composé **D**.

**12.** Composé à fonction(s) aldimine selon l'une quelconque des revendications 6-11, **caractérisé en ce que** le groupe réactif A est différent du groupe réactif A'.

**13.** Utilisation d'un composé à fonction(s) aldimine (I) selon l'une quelconque des revendications 1 à 12 comme source d'un aldéhyde de formule (IV)

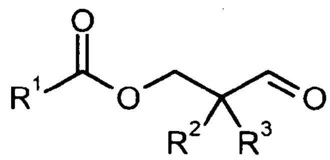

(IV)

**14.** Procédé d'hydrolyse d'un composé à fonction(s) aldimine selon l'une quelconque des revendications 1 à 12.

**15.** Procédé pour la réticulation d'un composé à fonction(s) aldimine selon l'une quelconque des revendications 1 à 12, où le produit de m et p de formule (I) vaut une valeur q ou moins, sous l'influence de l'eau.

**16.** Composition contenant un composé à fonction(s) aldimine selon l'une quelconque des revendications 1 à 12.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4469831 A
- US 4853454 A
- US 5087661 A
- WO 2004013088 A1
- US 4224417 A
- US 3493543 A
- US 3554974 A
- US 4108842 A
- US 4404379 A
- US 6136942 A


### In der Beschreibung aufgeführte Nicht-Patentliteratur


- **Houben-Weyl.** *Methoden der organischen Chemie,* vol. VIII, 516-528